(19) Europäisches Patentamt
European Patent Office
Office européen des brevets

(11) EP 4 186 942 B1

(12) **EUROPEAN PATENT SPECIFICATION**

(45) Date of publication and mention of the grant of the patent:
**25.12.2024 Bulletin 2024/52**

(21) Application number: **22804934.2**

(22) Date of filing: **16.05.2022**

(51) International Patent Classification (IPC):
***G02B 1/04*** (2006.01) ***C07C 43/23*** (2006.01)
***C07C 323/12*** (2006.01) ***C07D 209/86*** (2006.01)
***C07D 307/91*** (2006.01) ***C07D 333/76*** (2006.01)
***C08G 63/193*** (2006.01) ***C08G 63/672*** (2006.01)
***C08G 63/688*** (2006.01) ***C08G 64/16*** (2006.01)
***C08G 64/30*** (2006.01)

(52) Cooperative Patent Classification (CPC):
(C-Sets available)
**C07D 333/76; C07C 43/23; C07C 323/12; C07D 209/86; C07D 307/91; C08G 63/193; C08G 63/672; C08G 63/6886; C08G 64/1608; C08G 64/305; G02B 1/041** (Cont.)

(86) International application number:
**PCT/KR2022/006986**

(87) International publication number:
**WO 2022/245079 (24.11.2022 Gazette 2022/47)**

(54) **RESIN AND PREPARATION METHOD THEREFOR**

HARZ UND HERSTELLUNGSVERFAHREN DAFÜR

RÉSINE ET SON PROCÉDÉ DE PRÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.05.2021 KR 20210063418**

(43) Date of publication of application:
**31.05.2023 Bulletin 2023/22**

(73) Proprietor: **LG Chem, Ltd.**
**Seoul 07336 (KR)**

(72) Inventors:
- **JUNG, Min Suk**
  **Daejeon 34122 (KR)**
- **KIM, Kyeongmun**
  **Daejeon 34122 (KR)**
- **BAE, Jaesoon**
  **Daejeon 34122 (KR)**
- **SHIN, Hyeonah**
  **Daejeon 34122 (KR)**
- **YIM, Hye Jin**
  **Daejeon 34122 (KR)**
- **CHOI, Il Hwan**
  **Daejeon 34122 (KR)**
- **KIM, Kyeongmin**
  **Daejeon 34122 (KR)**
- **SHIN, Bora**
  **Daejeon 34122 (KR)**

(74) Representative: **Hoffmann Eitle**
**Patent- und Rechtsanwälte PartmbB**
**Arabellastraße 30**
**81925 München (DE)**

(56) References cited:
**WO-A1-2004/060962** **WO-A1-2007/050376**
**JP-A- S5 920 251** **KR-A- 20150 082 410**
**US-A- 4 994 547**

- **No further relevant documents disclosed**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**G02B 1/041, C08L 67/00;**
**G02B 1/041, C08L 69/00**

## Description

[Technical Field]

This application claims priority to and the benefit of Korean Patent Application No. 10-2021-0063418 filed in the Korean Intellectual Property Office on May 17, 2021.

The present invention relates to a resin and a method for preparing the same. More specifically, the present invention relates to a polyester or polycarbonate having a high refractive index and high transparency, and a method for preparing the same.

[Background Art]

The higher the refractive index of an optical material, the thinner the optical lens required to achieve the same level of correction. Accordingly, as the refractive index of the optical material is increased, a thinner and lighter lens can be manufactured, so that it is possible to make various devices, where lenses are used, smaller.

Generally, when the refractive index of an optical material is increased, there is a problem in that the Abbe's Number becomes low, and for use as an optical material, a certain level or higher of transparency is required.

WO 2004/060962 describes polycarbonates comprising repeating units derived from one or more of resorcinol, hydroquinone, methylhydroquinone, bisphenol A, and 4,4'-biphenol. The materials are prepared by the melt reaction of one or more of the aforementioned dihydroxy aromatic compounds with an ester-substituted diaryl carbonate such as bismethyl salicyl carbonate. In one embodiment, the polycarbonates may further comprise repeat units derived from a dihydroxy benzene of the following formula:

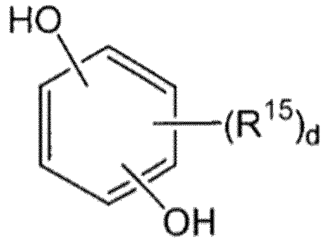

wherein $R^{15}$ is selected from a hydrogen atom, a halogen atom, a nitro group, a cyano group, a $C_{2-20}$ alkyl radical, a $C_{4-20}$ cycloalkyl radical or a $C_{4-20}$ aryl radical, and d is an integer from 1 to 4.

[Detailed Description of the Invention]

[Technical Problem]

The present invention has been made in an effort to provide a resin having a novel structure and a method for preparing the same.

Tthe present invention has also been made in an effort to provide a composition including a resin having a novel structure and a molded article prepared from the composition.

[Technical Solution]

The present invention provides a resin including a unit represented by the following Chemical Formula 1.

[Chemical Formula 1]

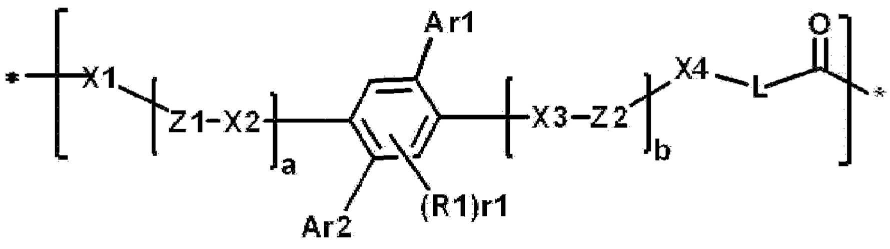

In Chemical Formula 1,

Ar1 and Ar2 are the same as or different from each other, and are each independently a substituted or unsubstituted aryl group; or a substituted or unsubstituted heteroaryl group,
R1 is hydrogen; or a substituted or unsubstituted alkyl group,
r1 is an integer from 0 to 2, and when r1 is 2, two R1's are the same as or different from each other,
L is a direct bond; or -CO-L'-,
L' is a substituted or unsubstituted arylene group,
X1 to X4 are the same as or different from each other, and are each independently O; or S,
Z1 and Z2 are the same as or different from each other, and are each independently a substituted or unsubstituted alkylene group; or a substituted or unsubstituted cycloalkylene group, and
a and b are the same as or different from each other, and are each independently an integer from 1 to 10, and when a and b are each 2 or higher, structures in each parenthesis are the same as or different from each other, and
* means a moiety linked to the main chain of the resin.

The present invention also provides a method for preparing the resin, the method including: polymerizing a composition for preparing a resin, which includes a compound represented by the following Chemical Formula 1a; and a polyester precursor or a polycarbonate precursor.

[Chemical Formula 1a]

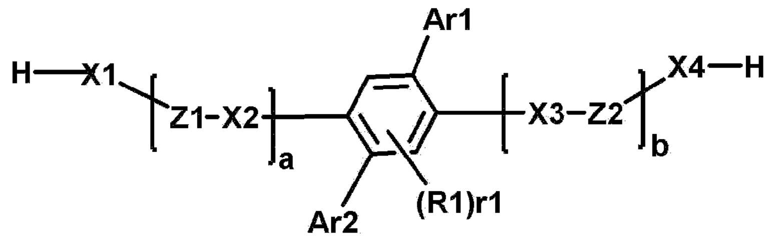

In Chemical Formula 1a, the definitions of Ar1, Ar2, R1, r1, X1 to X4, Z1, Z2, a and b are the same as the definitions in Chemical Formula 1.
Also disclosed herein is a compound represented by the following Chemical Formula 1a.

[Chemical Formula 1a]

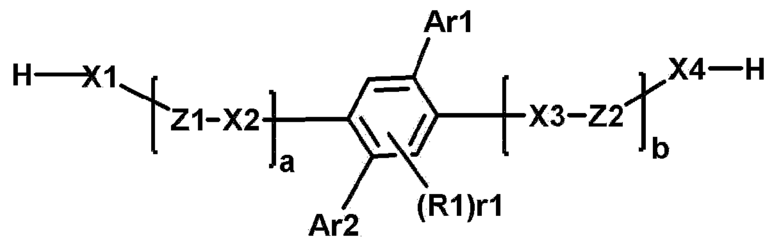

In Chemical Formula 1a,

Ar1 and Ar2 are the same as or different from each other, and are each independently an aryl group which is unsubstituted or substituted with a substituted or unsubstituted alkyl group, a substituted or unsubstituted aryl group, or a substituted or unsubstituted heteroaryl group; or a substituted or unsubstituted polycyclic heteroaryl group, when Ar1 and Ar2 are the same as each other and a phenyl group, the phenyl group is substituted with a substituted or unsubstituted alkyl group, a substituted or unsubstituted aryl group, or a substituted or unsubstituted heteroaryl group,
R1 is hydrogen; or a substituted or unsubstituted alkyl group,
r1 is an integer from 0 to 2, and when r1 is 2, two R1's are the same as or different from each other,
X1 to X4 are the same as or different from each other, and are each independently O; or S,

Z1 and Z2 are the same as or different from each other, and are each independently a substituted or unsubstituted alkylene group; or a substituted or unsubstituted cycloalkylene group, and

a and b are the same as or different from each other, and are each independently an integer from 1 to 10, and when a and b are each 2 or higher, structures in each parenthesis are the same as or different from each other. The present invention further provides a resin composition including the resin of the invention.

The present invention further provides a molded article including the resin composition according to the invention.

[Advantageous Effects]

The resin according to the present invention has a high refractive index and high transparency.

By using the resin according to the present invention, an excellent optical lens can be obtained.

[Best Mode]

Hereinafter, specific exemplary embodiments will be described in more detail.

The term "substitution" means that a hydrogen atom bonded to a carbon atom of a compound is changed into another substituent, and a position to be substituted is not limited as long as the position is a position at which the hydrogen atom is substituted, that is, a position at which the substituent may be substituted, and when two or more are substituted, the two or more substituents may be the same as or different from each other.

In the present specification, the term "substituted or unsubstituted" means being substituted with one or more substituents selected from the group consisting of a halogen; nitro ($NO_2$); nitrile group (CN) ; alkyl; cycloalkyl; aryl; an aryloxy group; an arylthio group; an alkylthio group; and heteroaryl, being substituted with a substituent to which two or more substituents among the exemplified substituents are linked, or having no substituent.

In the present specification, * means a binding moiety to another structure.

In the present specification, a cycloalkylene group may be a monocyclic or polycyclic cycloalkylene group. Specifically, the cycloalkylene group may be a cycloalkylene group having 3 to 20 carbon atoms; a monocyclic or polycyclic cycloalkylene group having 6 to 18 carbon atoms; or a monocyclic or polycyclic cycloalkylene group having 6 to 12 carbon atoms. More specifically, the cycloalkylene group may be a divalent group derived from an alicyclic hydrocarbon such as a cyclopentylene group, a cyclohexylene group, or a cycloheptylene group as the monocyclic cycloalkylene, and may be a divalent adamantane-diyl group, a divalent norbornane-diyl group as the polycyclic cycloalkylene group. Further, the cycloalkylene group may be unsubstituted or substituted one or more times with an alkyl group having 1 to 10 carbon atoms, an alkoxy group having 1 to 10 carbon atoms, or a halogen group.

In the present specification, the description on the cycloalkylene group may be applied, except that cycloalkyl group is a monovalent group rather than a divalent group.

In the present specification, an alkylene group may be a straight-chained or branched alkylene group as a divalent group derived from an aliphatic hydrocarbon having 1 to 30, 1 to 20, 1 to 10, or 1 to 5 carbon atoms. Specific examples of the alkylene group include a methylene group, an ethylene group, a propylene group, an n-propylene group, an isopropylene group, a butylene group, an n-butylene group, an isobutylene group, a tert-butylene group, a sec-butylene group, a 1-methyl-butylene group, a 1-ethylbutylene group, a pentylene group, an n-pentylene group, an isopentylene group, a neopentylene group, a tert-pentylene group, a hexylene group, an n-hexylene group, a 1-methylpentylene group, a 2-methylpentylene group, a 4-methyl-2-pentylene group, a 3,3-dimethylbutylene group, a 2-ethylbutylene group, a heptylene group, an n-heptylene group, a 1-methylhexylene group, an octylene group, an n-octylene group, a tert-octylene group, a 1-methylheptylene group, a 2-ethylhexylene group, a 2-propylpentylene group, an n-nonylene group, a 2,2-dimethylheptylene group, a 1-ethyl-propylene group, a 1,1-dimethyl-propylene group, an isohexylene group, a 2-methylpentylene group, a 4-methylhexylene group, and a 5-methylhexylene group.

In the present specification, the description on the straight-chained or branched alkylene group may be applied, except that a straight-chained or branched alkyl group is a monovalent group rather than a divalent group.

In the present specification, the alkyl group includes a straight-chained alkyl group and a branched alkyl group, unless otherwise limited.

In the present specification, an arylene group may be a monocyclic or polycyclic arylene group, and the number of carbon atoms thereof is not particularly limited, but is preferably 6 to 30, and may be 6 to 20. Specific examples of the monocyclic arylene group include a phenylene group, a biphenylylene group, and a terphenylylene group. When the arylene group is a polycyclic arylene group, the number of carbon atoms thereof is not particularly limited, but is preferably 10 to 30, and may be 10 to 20. Specific examples of the polycyclic arylene group include a naphthylene group, a divalent anthracene group, a divalent phenanthrene group, a divalent triphenylene group, a divalent pyrene group, a divalent perylene group, a divalent chrysene group, and a divalent fluorene group.

In the present specification, the description on the arylene group may be applied, except that the aryl group is

a monovalent group rather than a divalent group.

In the present specification, a heteroaryl group includes one or more atoms other than carbon, that is, one or more heteroatoms, and specifically, the heteroatom includes one or more atoms selected from the group consisting of O, N, Se, and S. The number of carbon atoms of the heteroaryl group is not particularly limited, but is preferably 1 to 30, and may be 1 to 20. The heteroaryl group may be monocyclic or polycyclic. Examples of the heteroaryl group include a thiophene group, a furan group, a dibenzofuran group, a dibenzothiophene group, a benzothiophene group, a pyrrole group, an imidazole group, a thiazole group, an oxazole group, an oxadiazole group, a pyridine group, a bipyridine group, a pyrimidine group, a triazine group, a triazole group, an acridine group, a pyridazine group, a pyrazine group, a quinoline group, a quinazoline group, a quinoxaline group, a phthalazine group, a pyridopyrimidine group, a pyridopyrazine group, a pyrazinopyrazine group, an isoquinoline group, an indole group, and a carbazole group.

In the present specification, the description on the heteroaryl group may be applied, except that the heteroarylene group is a divalent group rather than a monovalent group.

In the present specification, a halogen group is a fluoro group, a chloro group, a bromo group, or an iodo group.

In the present specification, the divalent aliphatic hydrocarbon group means the above-described alkylene group, and cycloalkylene group.

In the present specification, an alkoxy may be an alkoxy group having 1 to 10, or 1 to 5 carbon atoms. Specific examples of the alkoxy group include a methoxy group, an ethoxy group, an n-propoxy group, an isopropoxy group, an n-butoxy group, an isobutoxy group, a tert-butoxy group, a sec-butoxy group, a 1-methyl-butoxy group, a 1-ethyl-butoxy group, or a pentoxy group.

In the present specification, the description on the above-described cycloalkylene group may be applied, except that the aliphatic ring is a divalent group, and the description on the arylene group or heteroarylene group may be applied, except that the aromatic ring is a divalent group.

In the present specification, the aryloxy group may be represented by -ORo, and the description on the above-described aryl group is applied to Ro.

In the present specification, the arylthio group may be represented by -SRs1, and the description on the above-described aryl group is applied to Rs1.

In the present specification, the alkylthio group may be represented by -SRs2, and the description on the above-described alkyl group is applied to Rs2.

The present invention provides a resin including a unit represented by the following Chemical Formula 1.

[Chemical Formula 1]

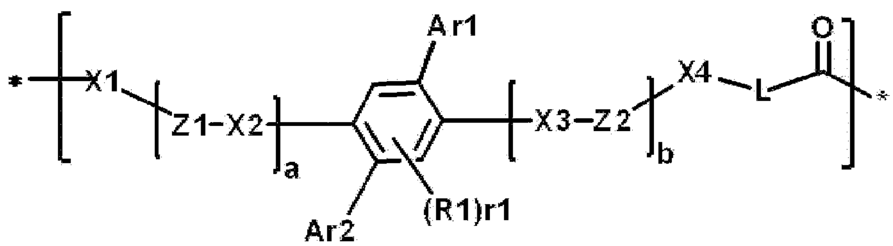

In Chemical Formula 1,

Ar1 and Ar2 are the same as or different from each other, and are each independently a substituted or unsubstituted aryl group; or a substituted or unsubstituted heteroaryl group,
R1 is hydrogen; or a substituted or unsubstituted alkyl group,
r1 is an integer from 0 to 2, and when r1 is 2, two R1's are the same as or different from each other,
L is a direct bond; or -CO-L'-,
L' is a substituted or unsubstituted arylene group,
X1 to X4 are the same as or different from each other, and are each independently O; or S,
Z1 and Z2 are the same as or different from each other, and are each independently a substituted or unsubstituted alkylene group; or a substituted or unsubstituted cycloalkylene group, and
a and b are the same as or different from each other, and are each independently an integer from 1 to 10, and when a and b are each 2 or higher, structures in each parenthesis are the same as or different from each other, and
* means a moiety linked to the main chain of the resin.

From the relationship form between the molecular structure and the refractive index, which is known by the Lorentz-Lorenz's formula, it can be seen that the refractive index of a material composed of molecules is increased by

increasing the electron density of the molecule and reducing the molecular volume.

The resin including the unit represented by Chemical Formula 1 has a small molecular volume and is excellent in the ability to pack, and thus may improve the refractive index of the resin because the core structure of Chemical Formula 1 is a phenylene group. Further, when Ar1 and Ar2 are a substituent rich in electrons such as a substituted or unsubstituted aryl group; or a substituted or unsubstituted heteroaryl group, the refractive index of the resin may be further improved by increasing the electron density of the structure represented by Chemical Formula 1.

In an exemplary embodiment of the present invention, Ar1 and Ar2 are the same as or different from each other, and are each independently a substituted or unsubstituted aryl group having 6 to 30 carbon atoms; or a substituted or unsubstituted heteroaryl group having 3 to 30 carbon atoms.

In an exemplary embodiment of the present invention, Ar1 and Ar2 are the same as or different from each other, and are each independently a substituted or unsubstituted aryl group having 6 to 20 carbon atoms; or a substituted or unsubstituted heteroaryl group having 3 to 20 carbon atoms.

In an exemplary embodiment of the present invention, Ar1 and Ar2 are the same as or different from each other, and are each independently a substituted or unsubstituted aryl group having 6 to 12 carbon atoms; or a substituted or unsubstituted heteroaryl group having 3 to 12 carbon atoms.

In an exemplary embodiment of the present invention, Ar1 and Ar2 are the same as or different from each other, and are each independently a substituted or unsubstituted phenyl group; a substituted or unsubstituted naphthyl group; a substituted or unsubstituted dibenzofuran group; a substituted or unsubstituted dibenzothiophene group; a substituted or unsubstituted carbazole group; a substituted or unsubstituted spirobifluorene group; or a substituted or unsubstituted fluorene group.

In an exemplary embodiment of the present invention, Ar1 and Ar2 are the same as or different from each other, and are each independently a phenyl group which is unsubstituted or substituted with a naphthyl group or a carbazole group; a naphthyl group which is unsubstituted or substituted with a naphthyl group; a dibenzofuran group; a dibenzothiophene group; a carbazole group which is unsubstituted or substituted with a phenyl group; a spirobifluorene group; or a fluorene group which is substituted with a phenyl group.

In an exemplary embodiment of the present invention, R1 is hydrogen; or a substituted or unsubstituted alkyl group having 1 to 30 carbon atoms.

In an exemplary embodiment of the present invention, R1 is hydrogen; or a substituted or unsubstituted alkyl group having 1 to 20 carbon atoms.

In an exemplary embodiment of the present invention, R1 is hydrogen; or a substituted or unsubstituted alkyl group having 1 to 10 carbon atoms.

In an exemplary embodiment of the present invention, R1 is hydrogen.

In an exemplary embodiment of the present invention, L is a direct bond.

In an exemplary embodiment of the present invention, L is -CO-L'-.

In an exemplary embodiment of the present invention, L' is a substituted or unsubstituted arylene group having 6 to 30 carbon atoms.

In an exemplary embodiment of the present invention, L' is a substituted or unsubstituted arylene group having 6 to 20 carbon atoms.

In an exemplary embodiment of the present invention, L' is a substituted or unsubstituted arylene group having 6 to 12 carbon atoms.

In an exemplary embodiment of the present invention, L' is a substituted or unsubstituted phenylene group.

In an exemplary embodiment of the present invention, L' is a phenylene group.

In an exemplary embodiment of the present invention, X1 to X4 are O.

In an exemplary embodiment of the present invention, X1 to X4 are S.

In an exemplary embodiment of the present invention, X1 and X4 are O.

In an exemplary embodiment of the present invention, X1 and X4 are S.

In an exemplary embodiment of the present invention, X2 and X3 are O.

In an exemplary embodiment of the present invention, X2 and X3 are S.

In an exemplary embodiment of the present invention, Z1 and Z2 are the same as or different from each other, and are each independently a substituted or unsubstituted alkylene group having 1 to 30 carbon atoms; or a substituted or unsubstituted cycloalkylene group having 3 to 30 carbon atoms.

In an exemplary embodiment of the present invention, Z1 and Z2 are the same as or different from each other, and are each independently a substituted or unsubstituted alkylene group having 1 to 20 carbon atoms; or a substituted or unsubstituted cycloalkylene group having 3 to 20 carbon atoms.

In an exemplary embodiment of the present invention, Z1 and Z2 are the same as or different from each other, and are each independently a substituted or unsubstituted alkylene group having 1 to 10 carbon atoms; or a substituted or unsubstituted cycloalkylene group having 3 to 10 carbon atoms.

In an exemplary embodiment of the present invention, Z1 and Z2 are the same as or different from each other,

and are each independently a substituted or unsubstituted alkylene group having 1 to 10 carbon atoms.

In an exemplary embodiment of the present invention, Z1 and Z2 are the same as or different from each other, and are each independently a substituted or unsubstituted methylene group; a substituted or unsubstituted ethylene group; a substituted or unsubstituted propylene group; or a substituted or unsubstituted hexylene group.

In an exemplary embodiment of the present invention, Z1 and Z2 are the same as or different from each other, and are each independently a methylene group; an ethylene group; a propylene group; or a hexylene group.

In an exemplary embodiment of the present invention, Z1 and Z2 are an ethylene group.

According to an exemplary embodiment of the present invention, the resin may have -OH; -SH; $-CO_2CH_3$; or $-OC_6H_5$ as an end group.

In an exemplary embodiment of the present invention, the resin has a weight average molecular weight of 10,000 g/mol to 200,000 g/mol, preferably 15,000 g/mol to 100,000 g/mol or 20,000 g/mol to 50,000 g/mol. The weight average molecular weight is more preferably 25,000 g/mol to 40,000 g/mol or 33,300 g/mol to 37,200 g/mol.

When the resin satisfies the above-described weight average molecular weight range, the resin may have optimum fluidity and processability.

In the present invention, the weight average molecular weights (Mws) of the resin and the oligomer used in the preparation thereof may be measured by gel permeation chromatograph (GPC) using a polystyrene (PS) standard using Agilent 1200 series. Specifically, the weight average molecular weights may be measured using an Agilent 1200 series device using a Polymer Laboratories PLgel MIX-B 300 mm length column, and in this case, the measurement temperature is 40°C, the used solvent is tetrahydrofuran (THF), and the flow rate is 1 mL/min. The sample of the resin or oligomer is each prepared at a concentration of 10 mg/10 mL, and then fed in an amount of 10 μL, and the Mw value is induced using a calibration curve formed using a polystyrene standard. In this case, nine types of polystyrene standard products with a molecular weight (g/mol) of 2,000 / 10,000 / 30,000 / 70,000 / 200,000 / 700,000 / 2,000,000 / 4,000,000 / 10,000,000 are used.

In an exemplary embodiment of the present invention, the resin may have a glass transition temperature (Tg) of 100°C to 160°C. Preferably, the glass transition temperature may be 111°C to 144°C. When the resin satisfies the above glass transition temperature range, the glass transition temperature is easily adjusted when a resin composition is prepared by mixing with a resin having excellent heat resistance and injectability and having a glass transition temperature different from the above-described range, so that the physical properties desired in the present invention may be satisfied.

The glass transition temperature (Tg) may be measured by a differential scanning calorimeter (DSC). Specifically, the glass transition temperature may be measured from a graph obtained by heating 5.5 mg to 8.5 mg of the resin sample to 270°C under a nitrogen atmosphere, then scanning the resin sample while heating the resin sample at a heating rate of 10°C/min during the second heating after cooling.

In an exemplary embodiment of the present invention, a refractive index of the resin, which is measured at a wavelength of 589 nm, is 1.64 to 1.71. The refractive index may be preferably 1.644 to 1.705. When the resin satisfies the above refractive index, a thin and light optical lens can be manufactured when the resin is applied to a molded article such as an optical lens.

In an exemplary embodiment of the present invention, the Abbe's Number of the resin, which is measured and calculated at a wavelength of 589 nm, 486nm, and 656nm may be 10 to 25. Preferably, the Abbe's Number may be 12 to 22. More preferably, the Abbe's Number may be 13.9 to 20.2. When the resin satisfies the above Abbe's Number range, there is an effect that the dispersion is decreased and the sharpness is increased when the resin is applied to a molded article such as an optical lens, while maintaining the high refractive index. The Abbe's Number may be obtained by the following Equation by measuring the refractive index ($n_D$, $n_F$, and $n_C$) at a wavelength of D (589 nm), F (486 nm), and C (656 nm), respectively at 20°C.

$$\text{Abbe's Number} = (n_D - 1)/(n_F - n_C)$$

The refractive index and the Abbe's Number may be measured from a film prepared by applying a solution prepared by dissolving the resin in a solvent to a silicon wafer by spin-coating, and may be measured by obtaining the result value according to the wavelength of light using an ellipsometer at 20°C for the applied film. The solution may be applied by the spin-coating at a rotation speed of 150 rpm to 300 rpm, and the applied film may have a thickness of 5 um to 20 um. The silicon wafer is not particularly limited, and any silicon wafer that can measure the refractive index and the Abbe's Number of the resin composition according to the present invention may be appropriately adopted. The solvent may be dimethylacetamide or 1,2-dichlorobenzene, and the solution may be prepared by dissolving the resin sample in an amount of 10 wt% based on the total weight of the solution.

The present invention also provides a method for preparing the resin, the method including: polymerizing a composition for preparing a resin, which includes a compound represented by the following Chemical Formula 1a; and

a polyester precursor or a polycarbonate precursor:

[Chemical Formula 1a]

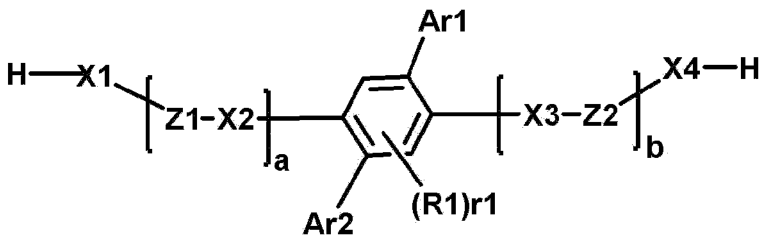

In Chemical Formula 1a, the definitions of Ar1, Ar2, R1, r1, X1 to X4, Z1, Z2, a and b are the same as the definitions in Chemical Formula 1.

An exemplary embodiment of the present invention preferably provides a method for preparing a resin, the method including: polymerizing a composition for preparing a resin, which includes the compound represented by Chemical Formula 1a and the polyester precursor.

An exemplary embodiment of the present invention preferably provides a method for preparing the resin, the method including: polymerizing a composition for preparing a resin, which includes the compound represented by Chemical Formula 1a and the polycarbonate precursor.

An exemplary embodiment of the present invention provides a composition for preparing a resin, which includes the compound represented by Chemical Formula 1a; and a polyester precursor or a polycarbonate precursor.

The composition for preparing a resin may further include a solvent.

The solvent may be, for example, diphenyl ether, dimethylacetamide or methanol, and any solvent applied in the art may be appropriately adopted.

The solvent may be included in an amount of 5 parts by weight to 60 parts by weight with respect to 100 parts by weight of the composition for preparing a resin.

The solvent may be included in an amount of preferably 5 parts by weight to 50 parts by weight, 10 parts by weight to 40 parts by weight or 10 parts by weight to 30 parts by weight with respect to 100 parts by weight of the composition for preparing a resin.

In an exemplary embodiment of the present invention, the compound represented by Chemical Formula 1a may be any one of the following compounds.

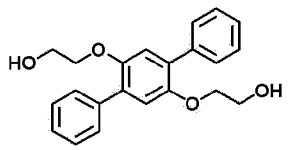
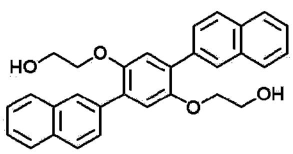
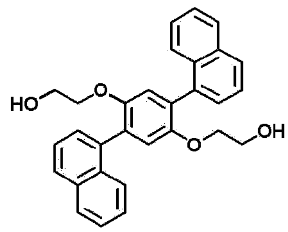

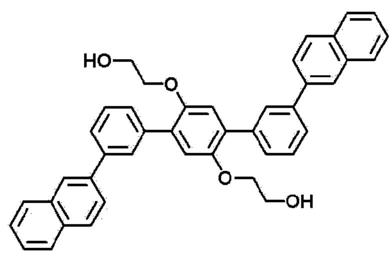
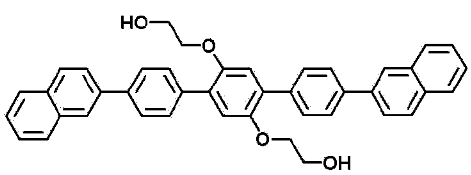

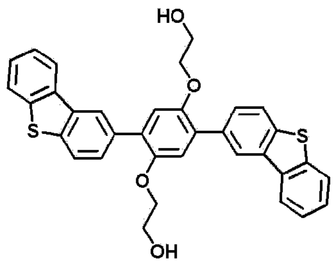

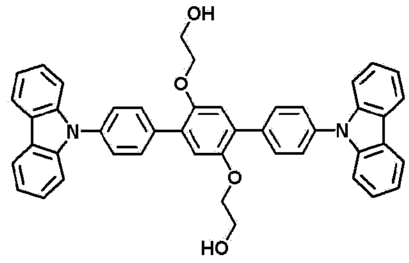

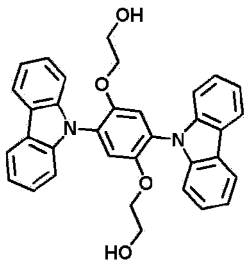

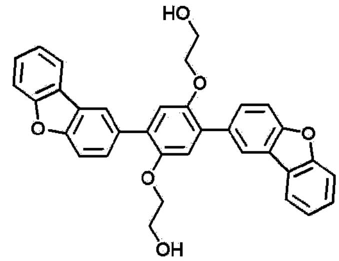

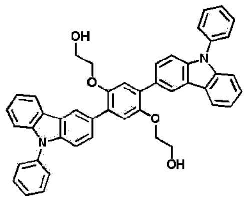

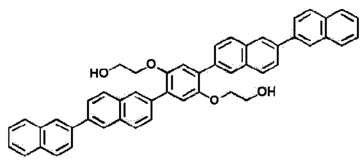

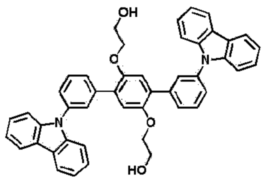

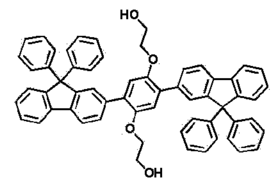

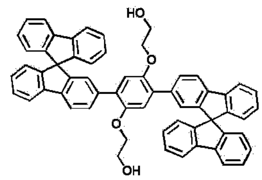

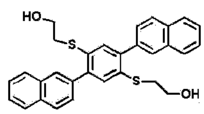

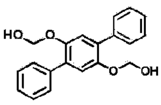

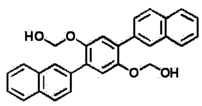

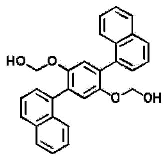

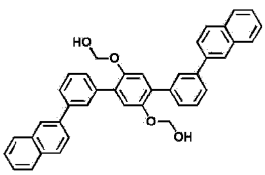

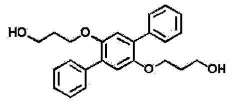

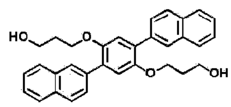

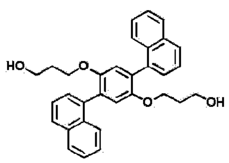

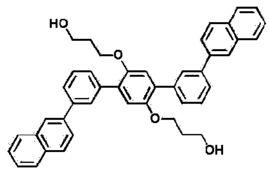

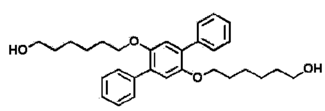

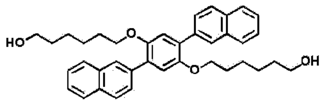

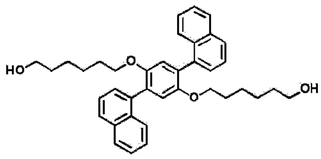

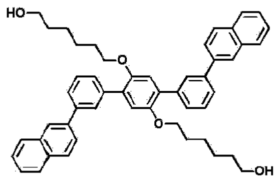

.

In an exemplary embodiment of the present invention, the compound represented by Chemical Formula 1a may be included in an amount of 1 part by weight to 99 parts by weight with respect to 100 parts by weight of the composition for preparing a resin.

The compound represented by Chemical Formula 1a may be included in an amount of preferably 1 to 60 parts by weight, 1 to 50 parts by weight, 1 to 40 parts by weight, 1 to 30 parts by weight or 1 to 20 parts by weight with respect to 100 parts by weight of the composition for preparing a resin.

In an exemplary embodiment of the present invention, the polyester precursor or the polycarbonate precursor may be included in an amount of 1 part by weight to 20 parts by weight with respect to 100 parts by weight of the composition for preparing a resin.

The polyester precursor or the polycarbonate precursor may be included in an amount of preferably 1 to 18 parts by weight, 1 to 16 parts by weight, 1 to 14 parts by weight, 1 to 12 parts by weight or 1 to 10 parts by weight with respect to 100 parts by weight of the composition for preparing a resin.

The compound represented by Chemical Formula 1a may be prepared by the following Reaction Scheme.

[Reaction Scheme]

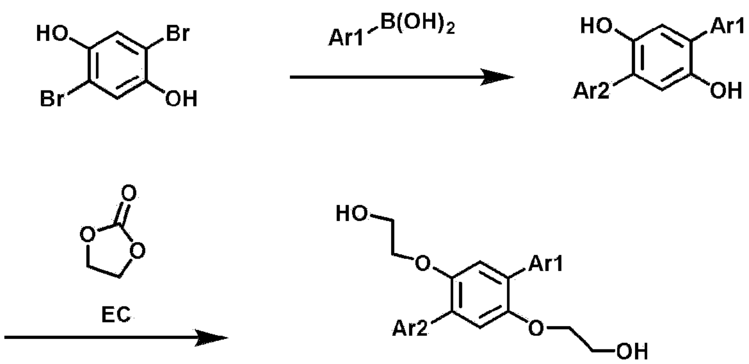


In the Reaction Scheme, the definition of the substituent is the same as the definition in the above-described Chemical Formula 1a.

Although the Reaction Scheme exemplifies a process of synthesizing a compound in which a specific substituent is bonded to a specific position, a unit corresponding to the range of Chemical Formula 1a may be synthesized by a synthesis method known in the art using a starting material and an intermediate material known in the art.

According to an exemplary embodiment of the present invention, the polyester precursor may be represented by the following Chemical Formula A, and the polycarbonate precursor may be represented by the following Chemical Formula B.

[Chemical Formula A]

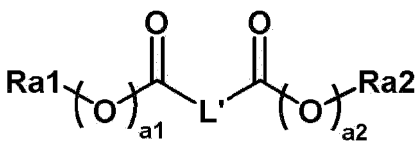

[Chemical Formula B]

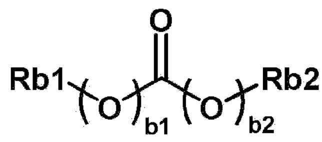

In Chemical Formulae A and B,

Ra1, Ra2, Rb1, and Rb2 are the same as or different from each other, and are each independently a halogen group; a substituted or unsubstituted alkyl group; or a substituted or unsubstituted aryl group,
L' is a substituted or unsubstituted arylene group, and
a1, a2, b1 and b2 are each 0 or 1.

In an exemplary embodiment of the present invention, Ra1, Ra2, Rb1, and Rb2 are the same as or different from each other, and are each independently a halogen group; a substituted or unsubstituted alkyl group having 1 to 30 carbon atoms; or a substituted or unsubstituted aryl group having 6 to 30 carbon atoms.

In an exemplary embodiment of the present invention, Ra1, Ra2, Rb1 and Rb2 are the same as or different from each other, and are each independently a halogen group; a substituted or unsubstituted alkyl group having 1 to 20 carbon atoms; or a substituted or unsubstituted aryl group having 6 to 20 carbon atoms.

In an exemplary embodiment of the present invention, Ra1, Ra2, Rb1 and Rb2 are the same as or different from each other, and are each independently a halogen group; a substituted or unsubstituted alkyl group having 1 to 10 carbon atoms; or a substituted or unsubstituted aryl group having 6 to 12 carbon atoms.

In an exemplary embodiment of the present invention, Ra1, Ra2, Rb1 and Rb2 are the same as or different from each other, and are each independently a chloro group; a substituted or unsubstituted methyl group; a substituted or unsubstituted ethyl group; or a substituted or unsubstituted phenyl group.

In an exemplary embodiment of the present invention, Ra1, Ra2, Rb1 and Rb2 are the same as or different from each other, and are each independently a chloro group; a methyl group; an ethyl group which is substituted with a hydroxyl group; or a phenyl group.

In an exemplary embodiment of the present invention, Ra1 and Ra2 are a chloro group.

In an exemplary embodiment of the present invention, Ra1 and Ra2 are a methyl group.

In an exemplary embodiment of the present invention, Ra1 and Ra2 is an ethyl group which is substituted with a hydroxyl group.

In an exemplary embodiment of the present invention, Rb1 and Rb2 are a chloro group.

In an exemplary embodiment of the present invention, Rb1 and Rb2 are a phenyl group.

In an exemplary embodiment of the present invention, L' is a substituted or unsubstituted arylene group having 6 to 30 carbon atoms.

In an exemplary embodiment of the present invention, L' is a substituted or unsubstituted arylene group having 6 to 20 carbon atoms.

In an exemplary embodiment of the present invention, L' is a substituted or unsubstituted arylene group having 6 to 12 carbon atoms.

In an exemplary embodiment of the present invention, L' is a substituted or unsubstituted phenylene group.

In an exemplary embodiment of the present invention, L' is a phenylene group.

In an exemplary embodiment of the present invention, a1 is 0.

In an exemplary embodiment of the present invention, a2 is 0.

In an exemplary embodiment of the present invention, b1 is 0.

In an exemplary embodiment of the present invention, b2 is 0.

In an exemplary embodiment of the present invention, a1 is 1.

In an exemplary embodiment of the present invention, a2 is 1.

In an exemplary embodiment of the present invention, b1 is 1.

In an exemplary embodiment of the present invention, b2 is 1.

The polycarbonate precursor serves to link an additional comonomer, if necessary, and other specific examples thereof which may be applied in addition to the compound represented by Chemical Formula B include phosgene, triphosgene, diphosgene, bromophosgene, dimethyl carbonate, diethyl carbonate, dibutyl carbonate, dicyclohexyl carbonate, ditolyl carbonate, bis(chlorophenyl) carbonate, m-cresyl carbonate, dinaphthyl carbonate, bis(diphenyl) carbonate, bishaloformate, or the like, and any one of them or a mixture of two or more thereof may be used.

The above-described unit of Chemical Formula 1 may be formed by polymerizing the compound represented by Chemical Formula 1a with the polyester precursor of Chemical Formula A or the polycarbonate precursor of Chemical Formula B.

In an exemplary embodiment of the present invention, it is preferred that the resin is polymerized from the compound represented by Chemical Formula 1a and the polyester precursor of Chemical Formula A.

The compound represented by Chemical Formula 1a may be used in an amount of 1 part by mol to 60 parts by mol with respect to 100 parts by mol of the entire monomer constituting the resin including the unit represented by Chemical Formula 1.

The polyester precursor represented by Chemical Formula A or the polycarbonate precursor represented by Chemical Formula B may be used in an amount of 50 parts by mol to 150 parts by mol with respect to 100 parts by mol of the entire monomer of the compound represented by Chemical Formula 1a, which constitutes the resin.

Also disclosed herein is a compound represented by the following Chemical Formula 1a.

[Chemical Formula 1a]

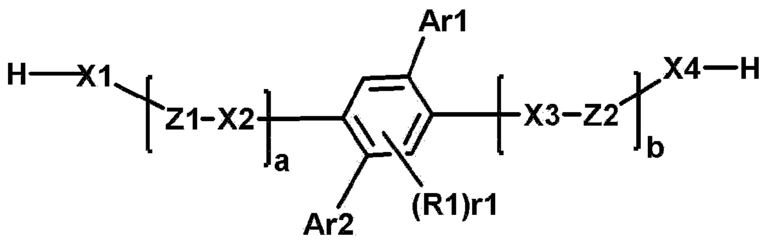

In Chemical Formula 1a,

Ar1 and Ar2 are the same as or different from each other, and are each independently an aryl group which is unsubstituted or substituted with a substituted or unsubstituted alkyl group, a substituted or unsubstituted aryl group, or a substituted or unsubstituted heteroaryl group; or a substituted or unsubstituted polycyclic heteroaryl group,
when Ar1 and Ar2 are the same as each other and a phenyl group, the phenyl group is substituted with a substituted or unsubstituted alkyl group, a substituted or unsubstituted aryl group, or a substituted or unsubstituted heteroaryl group,
R1 is hydrogen; or a substituted or unsubstituted alkyl group,
r1 is an integer from 0 to 2, and when r1 is 2, two R1's are the same as or different from each other,
X1 to X4 are the same as or different from each other, and are each independently O; or S,
Z1 and Z2 are the same as or different from each other, and are each independently a substituted or unsubstituted alkylene group; or a substituted or unsubstituted cycloalkylene group, and
a and b are the same as or different from each other, and are each independently an integer from 1 to 10, and when a and b are each 2 or higher, structures in each parenthesis are the same as or different from each other.

According to an exemplary embodiment of the present specification, Chemical Formula 1a is represented by the following Chemical Formula 1a-1.

[Chemical Formula 1a-1]

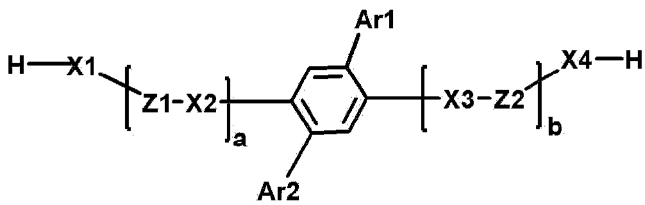

In Chemical Formula 1a-1, the definitions of Ar1, Ar2, X1 to X4, Z1, Z2, a and b are the same as the definitions in Chemical Formula 1a.

According to an exemplary embodiment of the present specification, Ar1 and Ar2 are the same as or different from each other, and are each independently an aryl group which is unsubstituted or substituted with an alkyl group, an aryl group, or a heteroaryl group; or a polycyclic heteroaryl group which is unsubstituted or substituted with an aryl group,

and when Ar1 and Ar2 are the same as each other and a phenyl group, the phenyl group is substituted with an alkyl group, an aryl group, or a heteroaryl group.

According to an exemplary embodiment of the present specification, Ar1 and Ar2 are the same as or different from each other, and are each independently a monocyclic or polycyclic aryl group having 6 to 30 carbon atoms, which is unsubstituted or substituted with a straight-chained or branched alkyl group having 1 to 30 carbon atoms, a monocyclic or polycyclic aryl group having 6 to 30 carbon atoms, or a monocyclic or polycyclic heteroaryl group having 2 to 30 carbon atoms; or a polycyclic heteroaryl group having 6 to 30 carbon atoms, which is unsubstituted or substituted with a monocyclic or polycyclic aryl group having 6 to 30 carbon atoms, and when Ar1 and Ar2 are the same as each other and a phenyl group, the phenyl group is substituted with a straight-chained or branched alkyl group having 1 to 30 carbon atoms, a monocyclic or polycyclic aryl group having 6 to 30 carbon atoms, or a monocyclic or polycyclic heteroaryl group having 2 to 30 carbon atoms.

According to an exemplary embodiment of the present specification, Ar1 and Ar2 are the same as or different from each other, and are each independently a phenyl group which is substituted with a monocyclic or polycyclic aryl group having 6 to 30 carbon atoms, or a monocyclic or polycyclic heteroaryl group having 3 to 30 carbon atoms; a naphthyl group which is unsubstituted or substituted with a monocyclic or polycyclic aryl group having 6 to 30 carbon atoms; a fluorene group which is substituted with a monocyclic or polycyclic aryl group having 6 to 30 carbon atoms; a spirobifluorene group; a dibenzofuran group; a dibenzothiophene group; or a carbazole group which is unsubstituted or substituted with a monocyclic or polycyclic aryl group having 6 to 30 carbon atoms.

According to an exemplary embodiment of the present specification, Ar1 and Ar2 are the same as or different from each other, and are each independently a phenyl group which is substituted with a naphthyl group or a carbazole group; a naphthyl group which is unsubstituted or substituted with a naphthyl group; a fluorene group which is substituted with a phenyl group; a spirobifluorene group; a dibenzofuran group; a dibenzothiophene group; or a carbazole group which is unsubstituted or substituted with a phenyl group.

An exemplary embodiment of the present specification provides a method for preparing a compound of the following Chemical Formula 1a, the method including: (s1) introducing the following Chemical Formula 1A, Ar1-$B(OH)_2$, and a first solvent;

(s2) introducing a first salt;
(s3) introducing a catalyst; and
(s4) introducing the following Chemical Formula 1B, a second salts, and a second solvent.

[Chemical Formula 1A]

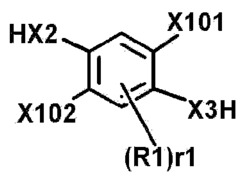

[Chemical Formula 1B]

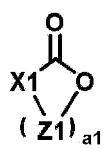

[Chemical Formula 1a]

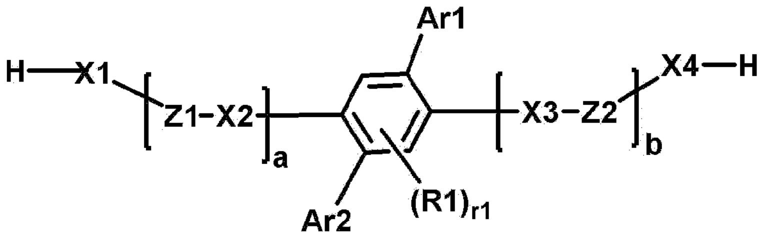

In Chemical Formulae 1A, 1B and 1a,

the definitions of Ar1, Ar2, R1, r1, X1 to X4, Z1, Z2, a and b are the same as those defined in Chemical Formula 1a, and X101 and X102 are the same as or different from each other, and are each independently a halogen group.

According to an exemplary embodiment of the present specification, X101 and X102 are a bromo group.

According to an exemplary embodiment of the present specification, the first solvent is selected among hexane, heptane, toluene, benzene, tetrahydrofuran, acetonitrile, dichloromethane, dichloroethane, trichloroethane, chloroform, dichloroform, nitromethane, dibromomethane, cyclopentanone, cyclohexanone, fluorobenzene, bromobenzene, chlorobenzene, xylene, mesitylene, dimethylacetamide, ethylacetate or any mixture thereof, and an organic solvent used in the related art may be used.

According to an exemplary embodiment of the present specification, the reaction temperature of Step (s1) is 50°C to 100°C, preferably 70°C to 90°C, and more preferably 80°C. When the reaction is performed within the above temperature range, there is an effect that a side reaction is suppressed and the reaction is promoted.

According to an exemplary embodiment of the present specification, the first salt may be potassium carbonate ($K_2CO_3$), sodium hydrogen carbonate ($NaHCO_3$), potassium hydrogen carbonate ($KHCO_3$) or any mixture thereof, and a salt used in the related art may be used.

According to an exemplary embodiment of the present specification, the reaction temperature of Step (s2) is 40°C to 80°C, preferably 45°C to 70°C, and more preferably 50°C to 60°C. When the reaction is performed within the above temperature range, there is an effect that a side reaction is suppressed because an over reaction does not occur.

According to an exemplary embodiment of the present specification, the catalyst is an organic metal catalyst.

According to an exemplary embodiment of the present specification, the catalyst includes a palladium-based metal catalyst.

According to an exemplary embodiment of the present specification, the catalyst is a palladium-based metal catalyst.

According to an exemplary embodiment of the present specification, the catalyst is preferably tetrakis(triphenylphosphine)palladium(0), [1,1'-bis(diphenylphosphino)ferrocene]dichloropalladium (II), bis(di-tert-butyl(4-dimethylaminophenyl)phosphine)dichloropalladium (II), or bis(tri-tert-butylphosphine)palladium(0) (BTP).

According to an exemplary embodiment of the present specification, the organic metal catalyst includes one palladium-based metal catalyst.

According to an exemplary embodiment of the present specification, the catalyst is bis(tri-tert-butylphosphine)palladium(0) (BTP).

According to an exemplary embodiment of the present specification, the catalyst is tetrakis(triphenylphosphine)palladium(0).

According to an exemplary embodiment of the present specification, the catalyst is [1,1'-bis(diphenylphosphino)ferrocene]dichloropalladium (II).

According to an exemplary embodiment of the present specification, the catalyst is bis(di-tert-butyl(4-dimethylaminophenyl)phosphine)dichloropalladium (II).

According to an exemplary embodiment of the present specification, the reaction temperature of Step (s3) is 50°C to 80°C, preferably 55°C to 70°C, and more preferably 60°C to 65°C. When the reaction is performed within the above temperature range, there is an effect that a side reaction is suppressed because an over reaction does not occur.

According to an exemplary embodiment of the specification, an intermediate purification step of purifying an intermediate product may be included. The intermediate purification step includes:

(s32) a quench and layer separation step;
(s33) a purification step;
(s34) a precipitation step; and
(s35) a concentration step.

According to an exemplary embodiment of the present specification, Step (s32) (quench and layer separation step) is a step of terminating the reaction by adding a reaction termination material, and separating the layers.

According to an exemplary embodiment of the present specification, as the reaction termination material of Step (s32), distilled water, an aqueous ammonium chloride solution, an aqueous sodium hydrogen carbonate solution, an aqueous HCl solution, an aqueous NaCl solution, and ethyl acetate may be used. Preferably, as the reaction termination material, an aqueous ammonium chloride solution, an aqueous sodium hydrogen carbonate solution, an aqueous NaCl solution, ethyl acetate, and distilled water are used.

In Step (s32), the order in which the reaction termination and layer separation are performed is not limited to a specific order. As an exemplary embodiment, reaction termination and layer separation may be simultaneously performed, and as another exemplary embodiment, the reaction is terminated by adding a reaction termination material, and then layers may be separated. However, the progression order is not limited to the above-described example.

According to an exemplary embodiment of the present specification, Step (s33)(purification step) is a step of purifying a layer-separated intermediate product. In the purification, column chromatography may be used. The column chromatography can be used without limitation as long as it is a method in the related art, and as an eluent of the column chromatography, methane, hexane (n-hexane), ethyl acetate, and dichloromethane may be used.

According to an exemplary embodiment of the present specification, Step (s34)(precipitation step) may be performed by a method known in the art. The step means a step of precipitating a purified intermediate product as a solid by selecting a solvent according to the solubility. A representative solvent is n-hexane.

According to an exemplary embodiment of the present specification, Step (s35)(concentration step) may be performed by a method known in the art. The step may be performed by evaporating the solvent using a vacuum rotary evaporator as a representative example. Through the (s35), the concentration is performed until the amount of the solvent becomes 5-fold compared to the theoretical yield (mass) of the product of Step (s3).

According to an exemplary embodiment of the present specification, Step (s4) includes a pre-treatment step. The pre-treatment step may be performed after Step (s3) and before Step (s4).

According to an exemplary embodiment of the present specification, the second salt may be potassium carbonate ($K_2CO_3$), sodium hydrogen carbonate ($NaHCO_3$), potassium hydrogen carbonate ($KHCO_3$) or any mixture thereof, and a salt used in the related art may be used.

According to an exemplary embodiment of the present specification, the second solvent is selected among hexane, heptane, toluene, benzene, tetrahydrofuran, acetonitrile, dichloromethane, dichloroethane, trichloroethane, chloroform, dichloroform, nitromethane, dibromomethane, cyclopentanone, cyclohexanone, fluorobenzene, bromobenzene, chlorobenzene, xylene, mesitylene, dimethylacetamide, ethylacetate or any mixture thereof, and an organic solvent used in the related art may be used.

According to an exemplary embodiment of the present specification, the reaction temperature of Step (s4) is 90°C to 130°C, preferably 100°C to 125°C, and more preferably 115°C to 120°C. When the reaction is performed within the above temperature range, there is an effect that a side reaction is suppressed and the reaction is promoted.

According to an exemplary embodiment of the present specification, a step of obtaining the compound of Chemical Formula 1a is further included after Step (s4).

The obtaining of the compound of Chemical Formula 1a according to an exemplary embodiment of the present specification includes:

(s41) a step of cooling a product of Step (s4);
(s42) quench;
(s43) a filtration step;
(s44) a purification step;
(s45) a precipitation step; and
(s46) an obtaining step.

According to an exemplary embodiment of the present specification, Step (s41) is performed by reducing the temperature to a temperature range of 30°C to 50°C. Preferably, Step (s41) may be performed by reducing the temperature to a temperature range of 45°C to 50°C. When the temperature range as described above is satisfied, the progress of the reaction may be terminated and heat generation may be prevented.

According to an exemplary embodiment of the present specification, the temperature reduction rate may be obtained such that the temperature gradient of the entire reaction system rather than a part of the reaction system is formed while gradually lowering the temperature of the reaction system. Specifically, the temperature may be reduced under a temperature reduction condition of 4°C/min to 7°C/min.

According to an exemplary embodiment of the present specification, Step (s42) (quench step) is a step of terminating the reaction by adding a reaction termination material.

According to an exemplary embodiment of the present specification, as the reaction termination material of

Step (s42), distilled water, an aqueous ammonium chloride solution, an aqueous sodium hydrogen carbonate solution, an aqueous KF (saturated) solution, an aqueous HCl solution, an aqueous NaCl (saturated) solution, chloroform, dichloromethane, and ethyl acetate may be used. Preferably, as the reaction termination material, distilled water is used.

According to an exemplary embodiment of the present specification, Step (s43)(filtration step) may be performed by a method known in the art. As a representative example, it is possible to use a method of inhaling air using an inhaler and filtering impurities including a desiccant, which are separated from a target product with a filter, or a method of removing other impurities using a solvent that does not dissolve a target product, but dissolves other impurities by utilizing the difference in solubility in a specific solvent.

According to an exemplary embodiment of the present specification, Step (s44)(purification step) is a step of purifying a layer-separated intermediate product. In the purification, column chromatography may be used. The column chromatography can be used without limitation as long as it is a method in the related art, and as an eluent of the column chromatography, methane, hexane (n-hexane), ethyl acetate, and dichloromethane may be used.

According to an exemplary embodiment of the present specification, Step (s45)(precipitation step) may be performed by a method known in the art. The step means a step of precipitating a purified intermediate product as a solid by selecting a solvent according to the solubility. A representative solvent is n-hexane.

It is possible to include a drying step before the (s46) obtaining step, and the drying step is a step of drying Chemical Formula 1a. The drying step may be performed by a method known in the art, representative examples thereof include vacuum oven drying, spray drying, and flash drying, and preferably, Chemical Formula 1a may be dried by vacuum oven drying.

According to an exemplary embodiment of the present specification, Step (s46) (obtaining step) is a step of obtaining the compound of Chemical Formula 1a which is precipitated (dried) in Step (s45).

For the polymerization of the resin according to the present invention, methods known in the art may be used.

It is preferred that the polymerization is performed by a melt polycondensation method.

In the melt polycondensation method, a catalyst may be further applied as needed using the composition for preparing a resin, and melt polycondensation may be performed under heating and further under normal pressure or reduced pressure while removing by-products by an ester exchange reaction. As the catalyst, a material generally applied in the art may be adopted.

Specifically, in the melt polycondensation method, it is preferred that the compound represented by Chemical Formula 1a; and the polyester precursor or the polycarbonate precursor are melted in a reaction vessel, and then a reaction is performed in a state where a by-product compound is allowed to stay.

In order to allow the by-product compound to stay, pressure may be controlled by closing the reaction device, or reducing pressure or increasing pressure.

The reaction time of this process is 20 minutes or more and 600 minutes or less, preferably 40 minutes or more and 450 minutes or less, and more preferably 60 minutes or more and 300 minutes or less.

In this case, when the by-product compound is distilled off immediately after being produced, a resin to be finally obtained has a small content of high molecular weight materials. However, when the by-produced monohydroxy compound is allowed to stay in the reaction vessel for a certain period of time, the finally obtained resin is obtained to have a large content of high molecular weight materials.

The melt polycondensation method may be performed continuously or in a batch manner. The reaction device used for performing the reaction may be a vertical type equipped with an anchor type impeller, a Maxblend impeller, a helical ribbon type impeller or the like, may be a horizontal type equipped with a paddle blade, a lattice blade, a spectacle-shaped blade or the like, and may be an extruder type equipped with a screw. In addition, it is desirable to use a reaction device that appropriately combines these reaction devices in consideration of the viscosity of the polymer.

In the method for preparing a resin used in the present invention, the catalyst may be removed or deactivated in order to maintain heat stability and hydrolysis stability after the completion of the polymerization reaction. A method of deactivating the catalyst by adding a known acidic material in the art may be preferably performed.

As the acidic material, for example, esters such as butyl benzoate, aromatic sulfonic acids such as p-toluenesulfonic acid; aromatic sulfonic acid esters such as butyl p-toluenesulfonate and hexyl p-toluenesulfonate; phosphoric acids such as phosphorous acid, phosphoric acid and phosphonic acid; phosphorous acid esters such as triphenyl phosphite, monophenyl phosphite, diphenyl phosphite, diethyl phosphite, di-n-propyl phosphite, di-n-butyl phosphite, di-n-hexyl phosphite, dioctyl phosphite and monooctyl phosphite, phosphoric acid esters such as triphenyl phosphate, diphenyl phosphate, monophenyl phosphate, dibutyl phosphate, dioctyl phosphate and monooctyl phosphate; phosphonic acids such as diphenylphosphonic acid, dioctylphosphonic acid and dibutylphosphonic acid; phosphonic acid esters such as diethyl phenylphosphonate; phosphines such as triphenylphosphine and bis(diphenylphosphino)ethane; boric acids such as boric acid and phenylboric acid; aromatic sulfonic acid salts such as dodecylbenzenesulfonic acid tetrabutylphosphonium salts; organic halides such as stearic acid chloride, benzoyl chloride and p-toluenesulfonic acid chloride; alkylsulfuric acids such as dimethylsulfuric acid; organic halides such as benzyl chloride are preferably used.

The acidic material may be used in an amount of 0.1 parts by mol to 5 parts by mol, preferably 0.1 parts by

mol to 1 part by mol with respect to 100 parts by mol of the catalyst.

When the amount of the acidic material is smaller than 0.1 parts by mol, the deactivation effect becomes insufficient, which is not preferred. Further, when the amount exceeds 5 parts by mol, the heat resistance of the resin deteriorates and the molded article is easily colored, which is not preferred.

After the catalyst is deactivated, a process of devolatilizing a low boiling point compound in the resin may be further performed under a pressure of 0.1 mmHg to 1 mmHg and at a temperature of 200°C to 350°C. In this process, a horizontal-type apparatus equipped with a stirring blade having excellent surface renewal ability such as a paddle blade, a lattice blade, and a spectacle-shaped blade, or a thin film evaporator is preferably used.

It is preferred that the content of foreign materials in the resin of the present invention is as small as possible, and filtration of a melting raw material, filtration of a catalyst solution are preferably performed.

The mesh of the filter used in the filtration is preferably 5 um or less, and more preferably 1 um or less. In addition, filtration of the produced resin using a polymer filter is preferably performed. The mesh of the polymer filter is preferably 100 um or less, and more preferably 30 um or less. Furthermore, a process of obtaining a resin pellet needs to be performed in a low-dust environment, and the environment is preferably Class 6 or lower, and more preferably Class 5 or lower.

Further, examples of a method of molding a molded article including the resin include compression molding, molds, roll processing, extrusion molding, and stretching, in addition to injection molding.

Another exemplary embodiment of the present invention provides a resin composition including the resin according to the above-described exemplary embodiments.

In an exemplary embodiment of the present invention, the resin may be included in an amount of 1 part by weight to 80 parts by weight based on 100 parts by weight of the resin composition.

In an exemplary embodiment of the present invention, the resin composition may further include a solvent. The solvent may be, for example, dimethylacetamide or 1,2-dichlorobenzene.

The solvent may be included in an amount of 20 parts by weight to 99 parts by weight based on 100 parts by weight of the resin composition.

The resin composition may include the resin further polymerized with an additional monomer in addition to the compound represented by Chemical Formula 1a. The additional monomer is not particularly limited, and a monomer generally applied in the art related to polyester or polycarbonate may be appropriately adopted as long as the main physical properties of the resin composition are not changed. The additional monomer may be used in an amount of 1 part by mol to 50 parts by mol with respect to 100 parts by mol of the entire monomer constituting the resin including the unit represented by Chemical Formula 1.

The resin composition may further include one or more selected from the group consisting of an additive, for example, an antioxidant, a plasticizer, an anti-static agent, a nucleating agent, a flame retardant, a lubricant, an impact modifier, a fluorescent brightener, a UV absorber, a pigment and a dye, if necessary, in addition to a resin including the unit represented by Chemical Formula 1.

The additive may be included in an amount of 1 part by weight to 99 parts by weight based on 100 parts by weight of the resin composition.

The type of antioxidant, plasticizer, anti-static agent, nucleating agent, flame retardant, lubricant, impact modifier, fluorescent brightener, UV absorber, pigment or dye is not particularly limited, and those applied in the art may be appropriately adopted.

Still another exemplary embodiment of the present invention provides a molded article including the resin composition according to the above-described exemplary embodiments.

In an exemplary embodiment of the present invention, the molded article may be prepared from the resin composition or a cured product thereof.

As an example of a method of preparing the molded article, it is possible to include mixing a resin including the above-described unit represented by Chemical Formula 1 and the additive well using a mixer, preparing the resulting mixture as a pellet by extrusion molding the mixture using an extruder, drying the pellet, and then injecting the pellet using an injection molding machine.

In an exemplary embodiment of the present invention, the molded article is an optical lens.

The optical lens is manufactured by using the resin, has a high refractive index and high transparency, and may be preferably applied to a camera.

In the case of camera modules in the art, a desired performance is adjusted through three or more or four or more lenses. Currently, in the case of optical lenses, particularly mobile camera modules, the first requirement is to reduce the thickness by molding the lens or using a high refractive index resin, and the present invention is focused on a resin having a high refractive index capable of satisfying such requirements. A lens combination in consideration of an ideal lens thickness and the molding difficulty of a molded article facilitates the use of a material having a high refractive index.

[Mode for Invention]

Hereinafter, the present invention will be exemplified in more detail through Examples.

Examples and Comparative Examples.

1. Synthesis of Monomer 1

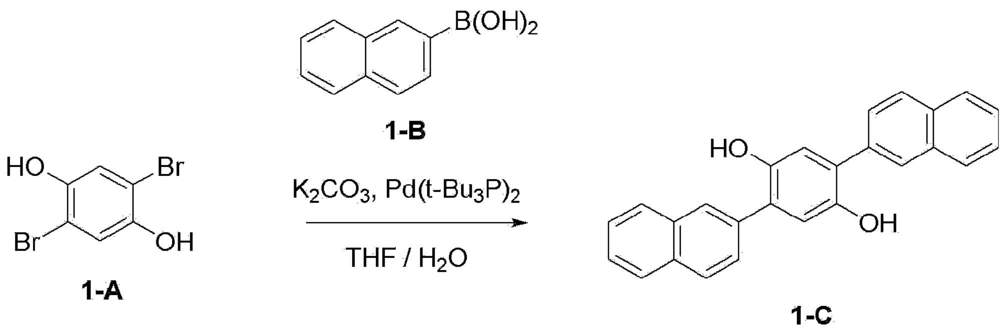


1-A

1-C

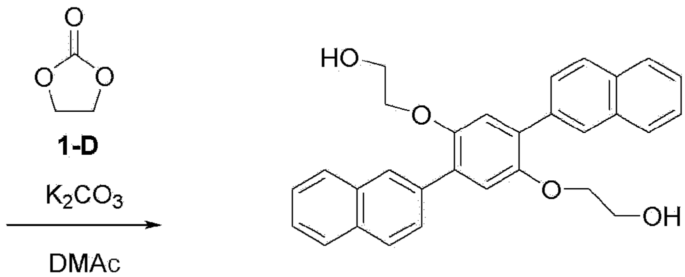


**Monomer 1**

1) Synthesis of Compound 1-C

10.0 g (37 mmol, 1.0 eq) of Compound 1-A and 13.16 g (77 mmol, 2.05 eq) of Compound 1-B were dissolved in 90 g of tetrahydrofuran (THF), and the resulting solution was stirred in an oil bath at 80°C for 30 minutes. After 15.48 g (112 mmol, 3.0 eq) of $K_2CO_3$ was dissolved in 100 mL of water, the solution was added dropwise thereto for 10 minutes while maintaining the internal temperature of the solution at 50°C or higher. 0.76 g (1.5 mmol, 0.04 eq) of a Pd(t-$Bu_3P)_2$ catalyst was added thereto at an internal temperature of 60°C. After being stirred for 1 hour, the mixture was washed with ethyl acetate (EA)/$H_2O$ to separate the organic layer, and the solvent was concentrated in vacuum. After purification by column chromatography through n-hexane (n-Hex) and dichloromethane (DCM), the resulting product was precipitated in n-hexane (n-Hex) to obtain Compound 1-C as a solid.

3) Synthesis of Monomer 1

9.0 g (25 mmol, 1.0 eq) of Compound 1-C, 5.47 g (62 mmol, 2.5 eq) of Compound 1-D, and 1.37 g (5 mmol, 0.40 eq) of $K_2CO_3$ were dissolved in 45 g of dimethylacetamide (DMAc), and the resulting solution was stirred in an oil bath at 120°C for 2 hours. After cooling, a solid was precipitated by adding water thereto, and then filtered. The obtained solid was purified by column chromatography through ethyl acetate (EA) and dichloromethane (DCM), and then precipitated in n-hexane (n-Hex) to obtain 6.7g of Monomer 1 as a white solid.
MS: $[M+H]^+$=450

2. Synthesis of Monomer 2

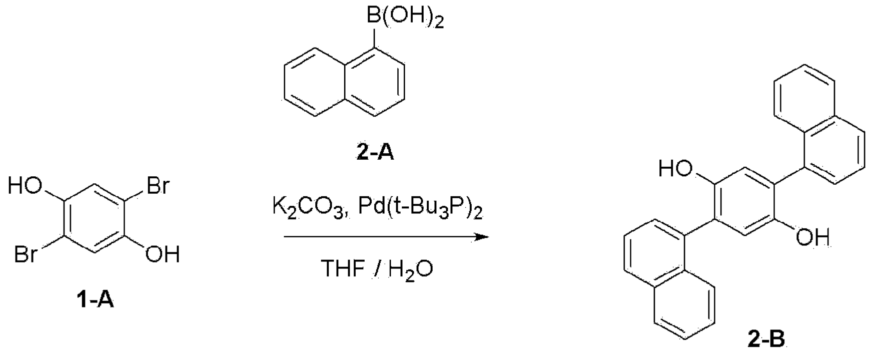


1-A 2-A 2-B

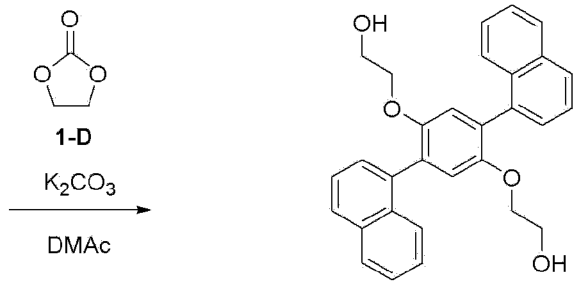


1-D

Monomer 2

Monomer 2 was obtained in the same manner as in the synthesis of Monomer 1, except that Compound 2-A was used instead of Compound 1-B.
MS: $[M+H]^+$=450

3. Synthesis of Monomer 3

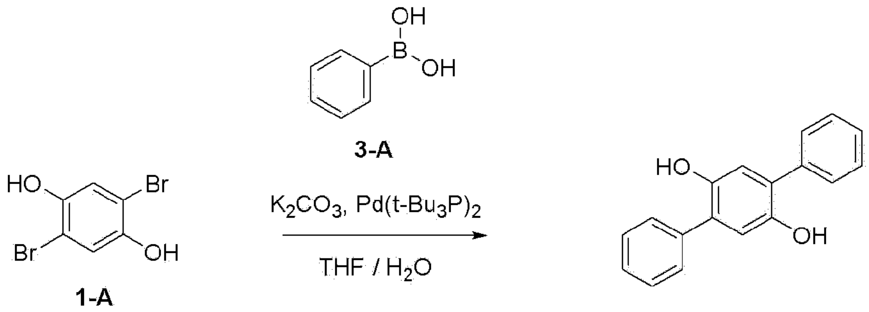


3-A

1-A

3-B

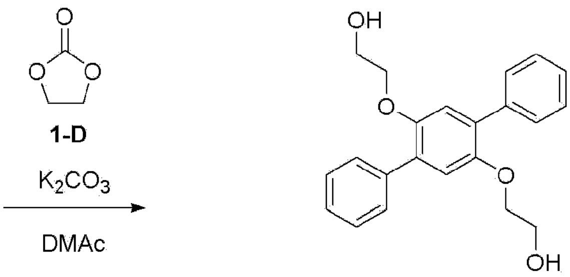


1-D

Monomer 3

Monomer 3 was obtained in the same manner as in the synthesis of Monomer 1, except that Compound 3-A was used instead of Compound 1-B.
MS: $[M+H]^+$=350

4. Synthesis of Monomer 4

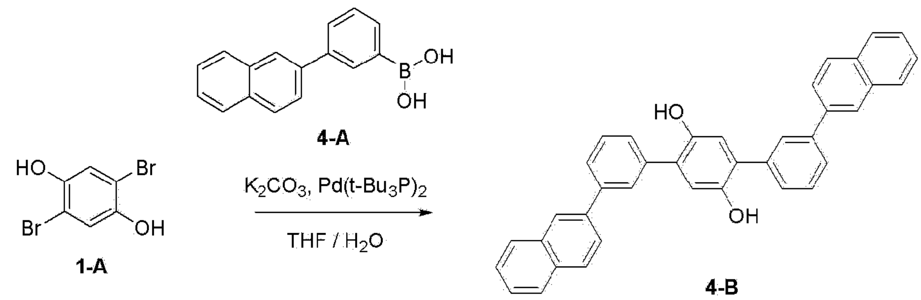


4-A

1-A

4-B

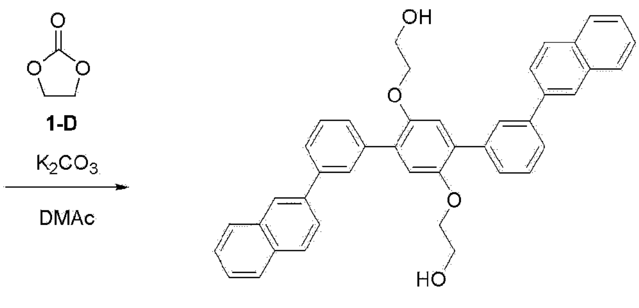


1-D

Monomer 4

Monomer 4 was obtained in the same manner as in the synthesis of Monomer 1, except that Compound 4-A was used instead of Compound 1-B.
MS: $[M+H]^+$=602

5. Synthesis of Monomer 5

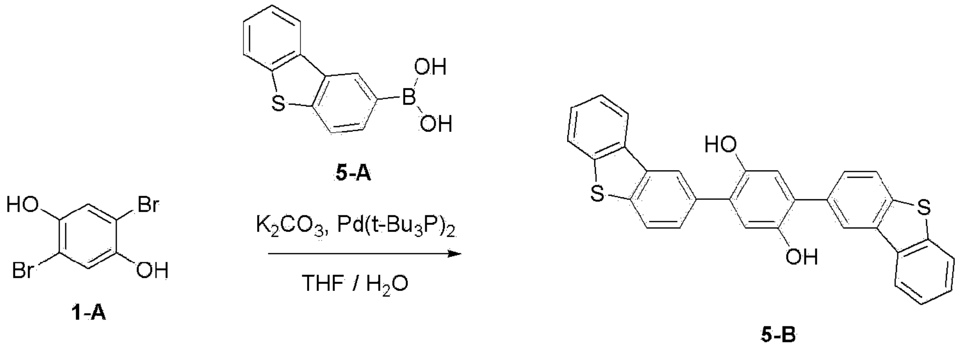

5-A
1-A
5-B

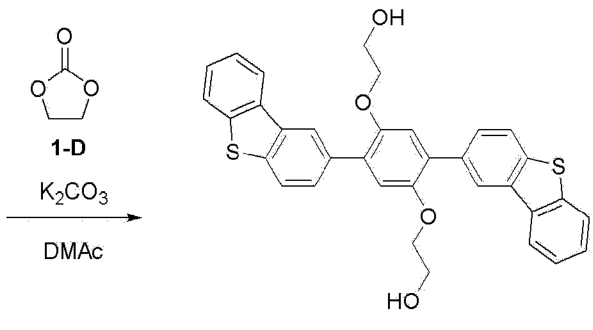

1-D

Monomer 5

Monomer 5 was obtained in the same manner as in the synthesis of Monomer 1, except that Compound 5-A was used instead of Compound 1-B.
MS: $[M+H]^+$=562

6. Synthesis of Monomer 6

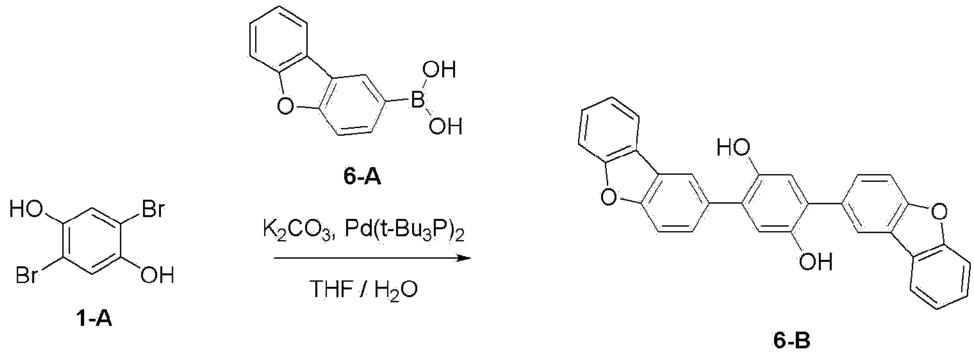


6-A

1-A

6-B

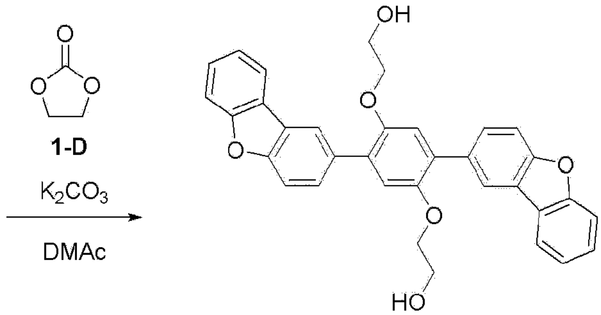


1-D

Monomer 6

Monomer 6 was obtained in the same manner as in the synthesis of Monomer 1, except that Compound 6-A was used instead of Compound 1-B.
MS: $[M+H]^+$=530

7. Synthesis of Monomer 7

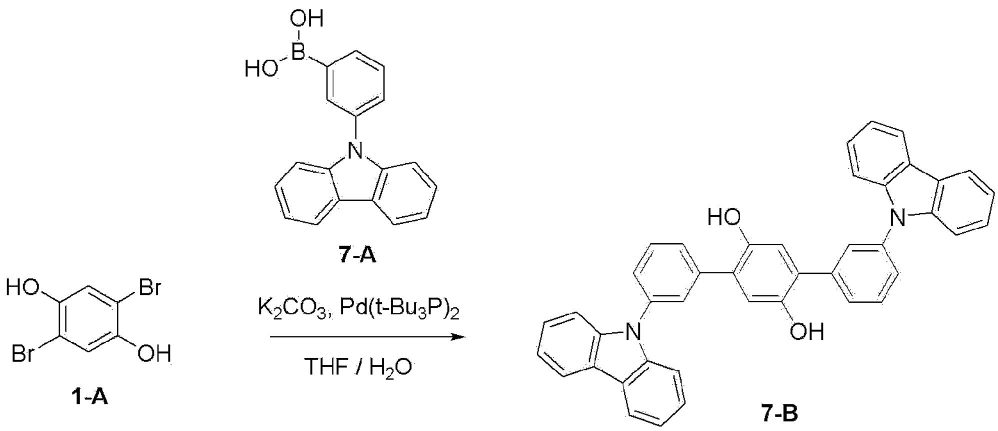


7-A

1-A

7-B

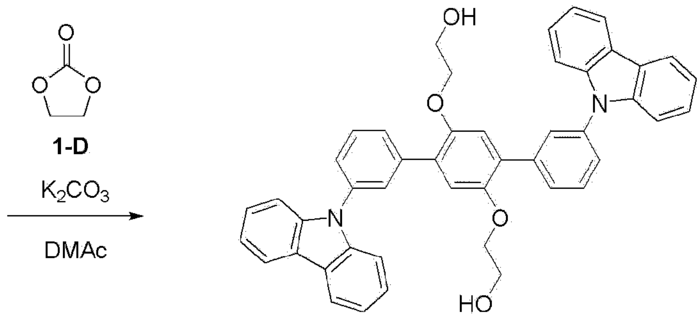


1-D

**Monomer 7**

Monomer 7 was obtained in the same manner as in the synthesis of Monomer 1, except that Compound 7-A was used instead of Compound 1-B.
MS: $[M+H]^+=680$

8. Synthesis of Monomer 8

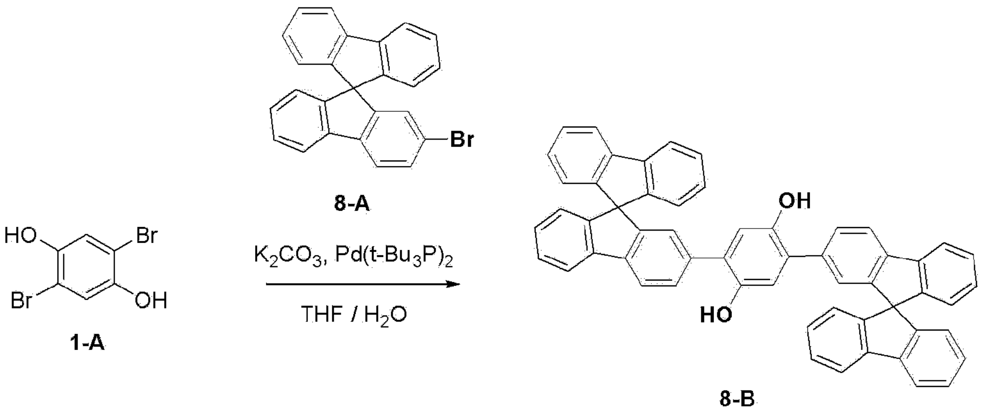


8-B

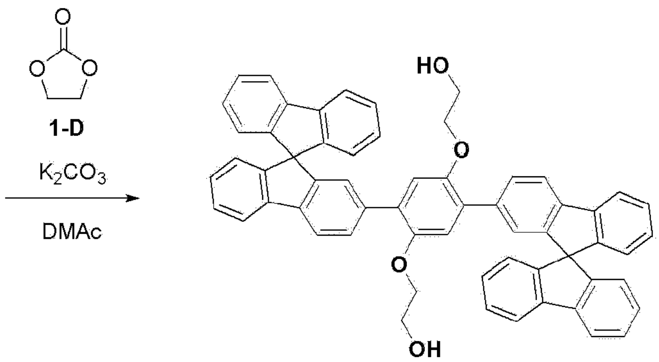


**Monomer 8**

Monomer 8 was obtained in the same manner as in the synthesis of Monomer 1, except that Compound 8-A was used instead of Compound 1-B.
MS: $[M+H]^+$=826

9. Synthesis of Monomer 9

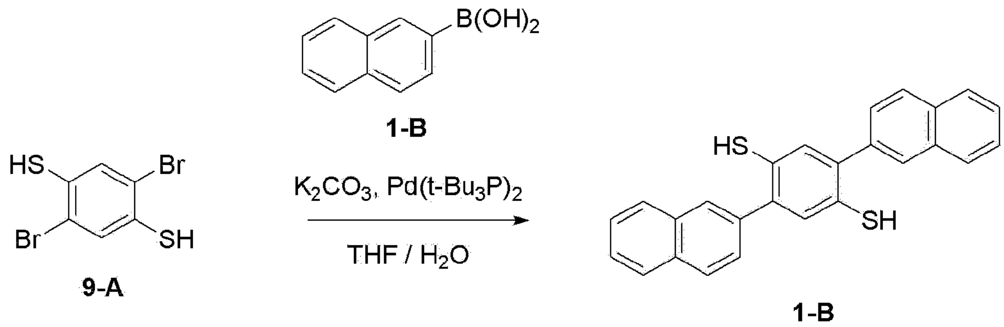


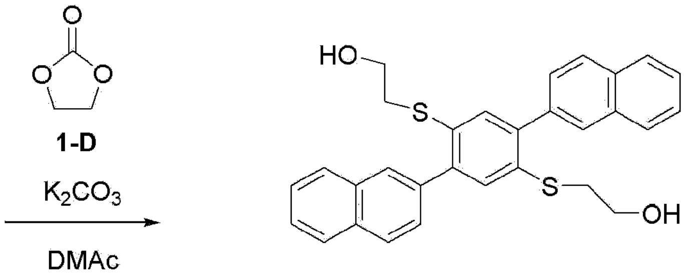


**Monomer 9**

Monomer 9 was obtained in the same manner as in the synthesis of Monomer 1, except that Compound 9-A was used instead of Compound 1-A.
MS: $[M+H]^+$=482

Monomer of Comparative Example C1

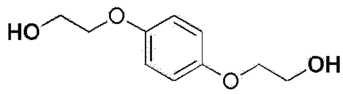

**C1**

The monomer of Comparative Example C1 was obtained from TCI Chemical or Alfa aesar.

Preparation of Resin (Polymer) 1

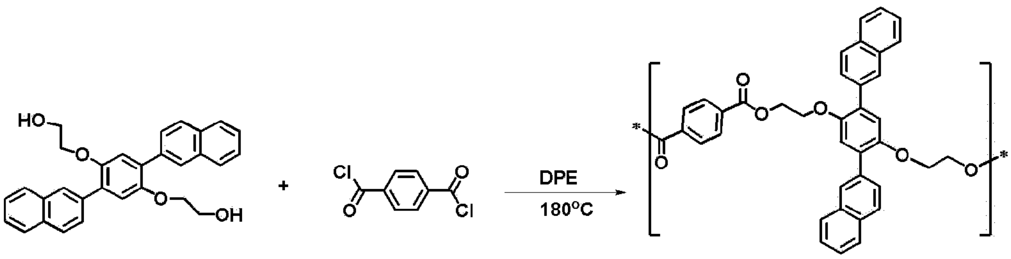


Monomer 1

Polymer 1

0.798 g (1.77 mmol, 1.0 eq) of Monomer 1 and 0.36 g (1.77 mmol, 1.0 eq) of terephthaloyl chloride were dissolved in 4.0 g of diphenyl ether (DPE), and the resulting solution was reacted in an oil bath at 180°C for 6 hours.

Chloric acid (HCl) gas was generated as the reaction proceeded, and a nitrogen substitution device and a chloric acid gas capturing device were installed to remove the gas. After the reaction, the resulting product was cooled to 100°C, 15 g of dimethylacetamide (DMAc) was added thereto, and the resulting mixture was precipitated through methanol (methylalcohol) to prepare Resin (Polymer) 1.

Preparation of Resins 2 to 9

Resins 2 to 9 were prepared in the same manner as in the method of preparing Resin 1, except that Monomers 2 to 9 were used instead of Monomer 1.

Preparation of Comparative Example Resin P1

Comparative Example Resin P1 was prepared in the same manner as in the method of preparing Resin 1, except that a monomer of Comparative Example C1 was used instead of Monomer 1.

Preparation of Resin (Polymer) 10

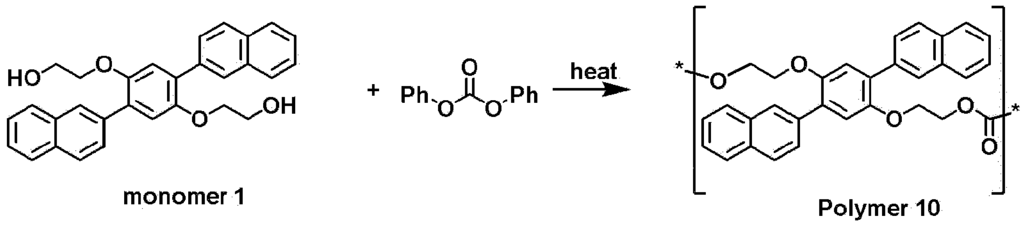


monomer 1

Polymer 10

44.15g (98 mmol) of Monomer 1 and 21.4 g (100 mmol) of diphenylcarbonate were melted and reacted at 250°C for 5 hours. As the reaction proceeded, phenol was generated as a by-product, and the degree of decompression was adjusted up to 1 Torr (1 Torr = 133.3224 Pa) to remove the phenol. After completion of the reaction, nitrogen was blown into the reactor to create a normal pressure atmosphere, and the polymerized molten resin was taken out, whereby Resin (Polymer) 10 as a polycarbonate was obtained.

Preparation of Resins 11 to 18

Resins 11 to 18 were prepared in the same manner as in the method of preparing Resin 10, except that Monomers 2 to 9 were used instead of Monomer 1.

Preparation of Comparative Example Resin P2

Comparative Example Resin P2 was prepared in the same manner as in the method of preparing Resin 10, except that a monomer of Comparative Example C1 was used instead of Monomer 1.

Experimental Example.

The molecular weight and molecular weight distribution of the polymerized resin sample were confirmed through gel permeation chromatography (GPC), and a thermogram was obtained using a differential scanning calorimeter (DSC) to investigate the thermal characteristics. After a film was formed to measure the refractive index and the Abbe's Number, a result value according to the wavelength of light was obtained using an ellipsometer.

For the molecular weight through gel permeation chromatography (GPC), results were obtained by injecting a solution produced using tetrahydrofuran (THF, stabilized with butylated hydroxytoluene (BHT)) as a solvent, dissolving the resin sample in tetrahydrofuran at a concentration of 1.0 mg/1 ml, filtering the dissolved resin sample with a syringe filter, and measuring the molecular weight at 40°C, and the results are shown in the following Table 1. A Waters RI detector was used, and two Agilent PLgel MIXED-B columns were used.

A differential scanning calorimeter (DSC) was measured to determine the glass transition temperature (Tg) of the resin. A glass transition temperature (Tg) was obtained on a graph obtained by heating a 5.5 mg to 8.5 mg of the resin sample to 270°C under $N_2$ flow, cooling the resin sample, and then scanning the resin sample while heating the

resin sample at a heating rate of 10°C/min during the second heating, and the glass transition temperature (Tg) is shown in the following Table 1.

In order to measure the refractive index and Abbe's Number of the resin, a polymer solution prepared by dissolving a resin powder sample obtained by polymerization in a solvent dimethylacetamide in an amount of 10 wt% based on the total weight of the polymer solution was applied onto a silicon wafer at a rotation speed of 220 rpm by spin coating to form a film having a thickness of 20 um, and then the resulting values according to the wavelength of light were obtained at 20°C using an ellipsometer, and are shown in the following Table 1. Specifically, the refractive index was measured at a wavelength of 589 nm, and for the Abbe's Number, an Abbe's Number was obtained by the following Equation by measuring the refractive index ($n_D$, $n_F$, and $n_C$) at a wavelength of D (589 nm), F (486 nm), and C (656 nm), respectively.

$$\text{Abbe's Number} = (n_D - 1)/(n_F - n_C)$$

[Table 1]

| | Resin | Mn (g/mol) | Mw (g/mol) | Tg (°C) | Refractive index (589nm) | Abbe's Number |
|---|---|---|---|---|---|---|
| Example 1 | 1 | 21900 | 35900 | 121 | 1.699 | 14.5 |
| Example 2 | 2 | 22400 | 36200 | 119 | 1.664 | 17.6 |
| Example 3 | 3 | 23100 | 37100 | 111 | 1.644 | 19.5 |
| Example 4 | 4 | 21600 | 33500 | 131 | 1.692 | 15.1 |
| Example 5 | 5 | 23300 | 36200 | 122 | 1.668 | 18.1 |
| Example 6 | 6 | 23500 | 36600 | 121 | 1.664 | 18.2 |
| Example 7 | 7 | 19800 | 33300 | 138 | 1.705 | 13.9 |
| Example 8 | 8 | 24500 | 37200 | 144 | 1.701 | 14.1 |
| Example 9 | 9 | 21400 | 34100 | 124 | 1.701 | 16.8 |
| Example 10 | 10 | 22100 | 36100 | 120 | 1.695 | 14.7 |
| Example 11 | 11 | 23300 | 37100 | 119 | 1.661 | 17.6 |
| Example 12 | 12 | 22100 | 33600 | 107 | 1.640 | 20.2 |
| Example 13 | 13 | 21500 | 33300 | 128 | 1.685 | 15.4 |
| Example 14 | 14 | 23800 | 36700 | 122 | 1.659 | 18.3 |
| Example 15 | 15 | 23300 | 36500 | 119 | 1.652 | 18.6 |
| Example 16 | 16 | 21100 | 35300 | 139 | 1.699 | 14.1 |
| Example 17 | 17 | 24400 | 37000 | 141 | 1.691 | 14.3 |
| Example 18 | 18 | 22200 | 34200 | 122 | 1.698 | 16.9 |
| Comparative Example 1 | P1 | 27800 | 39400 | 94 | 1.611 | 24.2 |
| Comparative Example 2 | P2 | 28100 | 39900 | 93 | 1.607 | 24.3 |

In Table 1, Mn means the number average molecular weight, Mw means the weight average molecular weight, and the refractive index is a value measured at a wavelength of 589 nm.

According to Table 1, the resin according to an exemplary embodiment of the present invention includes the unit represented by Chemical Formula 1, and in particular, the core structure of Chemical Formula 1 is a phenylene group, so that it could be confirmed that the refractive index of the resin was improved because the molecular volume is small and the ability to be packed is excellent. Furthermore, it could be confirmed that when Ar1 and Ar2 are a substituent rich in electrons such as a substituted or unsubstituted aryl group; or a substituted or unsubstituted heteroaryl group, the refractive index of the resin may be further improved by increasing the electron density of the structure represented by Chemical Formula 1.

Currently, in the case of optical lenses, particularly mobile camera modules, the first requirement is to reduce

the thickness by molding the lens or using a high refractive index resin. It could be confirmed that the resin according to an exemplary embodiment of the present specification is a highly utilized resin as an optical material because Examples 1 to 18 have a refractive index capable of satisfying the above requirement rather than Comparative Examples 1 to 2.

## Claims

1. A resin comprising a unit represented by the following Chemical Formula 1:

[Chemical Formula 1]

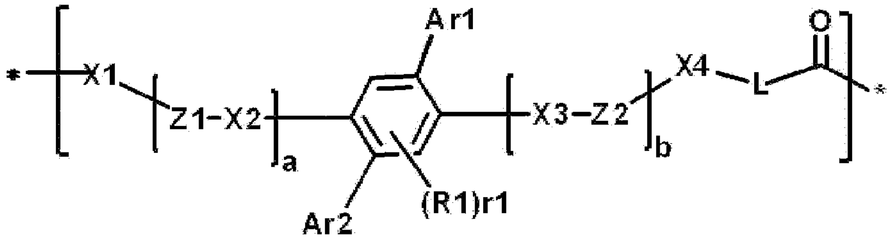

in Chemical Formula 1,

Ar1 and Ar2 are the same as or different from each other, and are each independently a substituted or unsubstituted aryl group; or a substituted or unsubstituted heteroaryl group,
R1 is hydrogen; or a substituted or unsubstituted alkyl group,
r1 is an integer from 0 to 2, and when r1 is 2, two R1's are the same as or different from each other,
L is a direct bond; or -CO-L'-,
L' is a substituted or unsubstituted arylene group,
X1 to X4 are the same as or different from each other, and are each independently O; or S,
Z1 and Z2 are the same as or different from each other, and are each independently a substituted or unsubstituted alkylene group; or a substituted or unsubstituted cycloalkylene group, and
a and b are the same as or different from each other, and are each independently an integer from 1 to 10, and when a and b are each 2 or higher, structures in each parenthesis are the same as or different from each other, and
* means a moiety linked to the main chain of the resin.

2. The resin of claim 1, wherein R1 is hydrogen.

3. The resin of claim 1, wherein Ar1 and Ar2 are the same as or different from each other, and are each independently a substituted or unsubstituted aryl group having 6 to 12 carbon atoms; or a substituted or unsubstituted heteroaryl group having 3 to 12 carbon atoms.

4. The resin of claim 1, wherein Z1 and Z2 are the same as or different from each other, and are each independently a substituted or unsubstituted alkylene group having 1 to 10 carbon atoms.

5. The resin of claim 1, wherein X1 to X4 are O.

6. The resin of claim 1, wherein the resin has a weight average molecular weight of 10,000 g/mol to 200,000 g/mol, measured as indicated in the description.

7. The resin of claim 1, wherein the resin has a glass transition temperature (Tg) of 100°C to 160°C, measured as indicated in the description.

8. The resin of claim 1, a refractive index of the resin, which is measured at a wavelength of 589 nm is 1.64 to 1.71 as indicated in the description.

9. A method for preparing the resin of any one of claims 1 to 8, the method comprising:

polymerizing a composition for preparing a resin, which comprises a compound represented by the following

Chemical Formula 1a; and a polyester precursor or a polycarbonate precursor:

[Chemical Formula 1a]

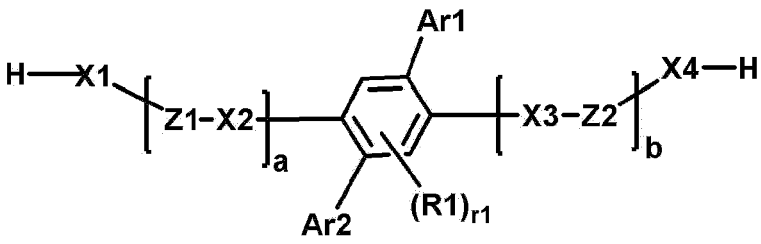

in Chemical Formula 1a, the definitions of Ar1, Ar2, R1, r1, X1 to X4, Z1, Z2, a and b are the same as the definitions in Chemical Formula 1.

**10.** The method of claim 9, wherein the polyester precursor is represented by the following Chemical Formula A, and the polycarbonate precursor is represented by the following Chemical Formula B:

[Chemical Formula A]

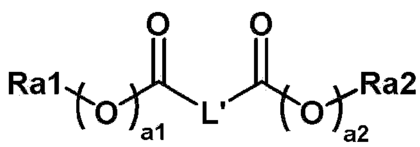

[Chemical Formula B]

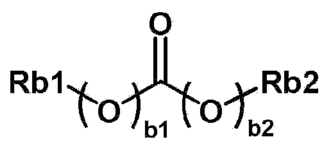

in Chemical Formulae A and B,

Ra1, Ra2, Rb1, and Rb2 are the same as or different from each other, and are each independently a halogen group; a substituted or unsubstituted alkyl group; or a substituted or unsubstituted aryl group,
L' is a substituted or unsubstituted arylene group, and
a1, a2, b1 and b2 are each 0 or 1.

**11.** A compound represented by the following Chemical Formula 1a:

[Chemical Formula 1a]

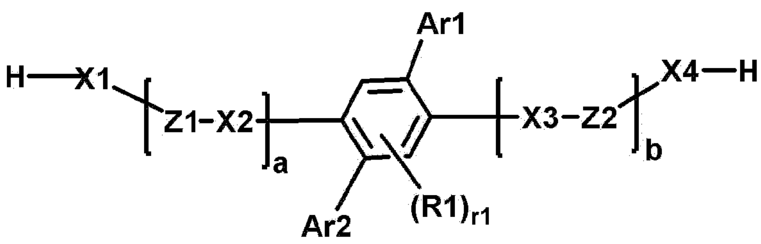

in Chemical Formula 1a,

Ar1 and Ar2 are the same as or different from each other, and are each independently an aryl group which is unsubstituted or substituted with a substituted or unsubstituted alkyl group, a substituted or unsubstituted aryl group, or a substituted or unsubstituted heteroaryl group; or a substituted or unsubstituted polycyclic heteroaryl group,
when Ar1 and Ar2 are the same as each other and a phenyl group, the phenyl group is substituted with a substituted or unsubstituted alkyl group, a substituted or unsubstituted aryl group, or a substituted or unsubstituted heteroaryl group,
R1 is hydrogen; or a substituted or unsubstituted alkyl group,
r1 is an integer from 0 to 2, and when r1 is 2, two R1's are the same as or different from each other,
X1 to X4 are the same as or different from each other, and are each independently O; or S,
Z1 and Z2 are the same as or different from each other, and are each independently a substituted or unsubstituted alkylene group; or a substituted or unsubstituted cycloalkylene group, and
a and b are the same as or different from each other, and are each independently an integer from 1 to 10, and when a and b are each 2 or higher, structures in each parenthesis are the same as or different from each other.

**12.** A resin composition comprising the resin of any one of claims 1 to 8.

**13.** A molded article comprising the resin composition of claim 12.

**14.** The molded article of claim 13, wherein the molded article is an optical lens.

## Patentansprüche

**1.** Harz, umfassend eine durch die folgende chemische Formel 1 dargestellte Einheit:

[Chemische Formel 1]

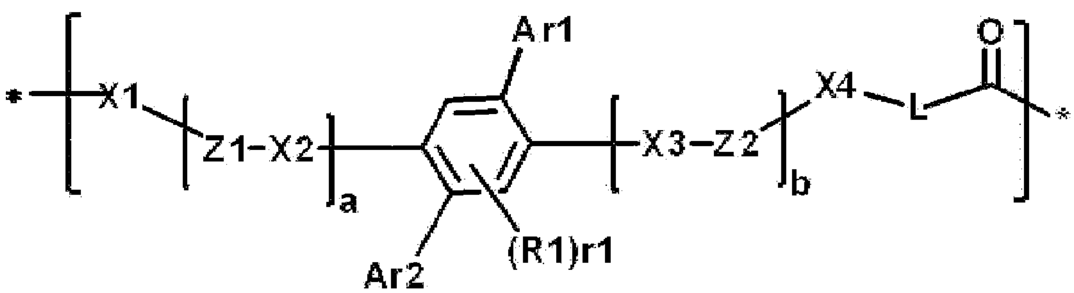

worin in der chemischen Formel 1

Ar1 und Ar2 gleich oder verschieden sind und jeweils individuell eine substituierte oder unsubstituierte Arylgruppe; oder eine substituierte oder unsubstituierte Heteroarylgruppe sind,
R1 Wasserstoff; oder eine substituierte oder unsubstituierte Alkylgruppe ist,
r1 eine ganze Zahl von 0 bis 2 ist, und wenn r1 2 ist, zwei R1 gleich oder verschieden sind,
L eine direkte Bindung; oder -CO-L'- ist,
L' eine substituierte oder unsubstituierte Arylengruppe ist,
X1 bis X4 gleich oder verschieden sind und jeweils unabhängig O; oder S sind,
Z1 und Z2 gleich oder verschieden sind und jeweils unabhängig eine substituierte oder unsubstituierte Alkylengruppe; oder eine substituierte oder unsubstituierte Cycloalkylengruppe sind, und
a und b jeweils gleich oder verschieden sind und jeweils unabhängig eine ganze Zahl von 1 bis 10 sind, und wenn a und b jeweils 2 oder höher sind, die Strukturen in jeder Klammer gleich oder voneinander verschieden sind und
* einen Rest darstellt, der an die Hauptkette des Harzes gebunden ist.

**2.** Harz gemäß Anspruch 1, worin R1 Wasserstoff ist.

3. Harz gemäß Anspruch 1, worin Ar1 und Ar2 gleich oder verschieden sind und jeweils unabhängig eine substituierte oder unsubstituierte Arylgruppe mit 6 bis 12 Kohlenstoffatomen; oder eine substituierte oder unsubstituierte Heteroarylgruppe mit 3 bis 12 Kohlenstoffatomen sind.

4. Harz gemäß Anspruch 1, worin Z1 und Z2 gleich oder voneinander verschieden sind und jeweils unabhängig eine substituierte oder unsubstituierte Alkylengruppe mit 1 bis 10 Kohlenstoffatomen sind.

5. Harz gemäß Anspruch 1, worin X1 bis X4 O sind.

6. Harz gemäß Anspruch 1, worin das Harz ein gewichtsgemitteltes Molekulargewicht von 10.000 g/mol bis 200.000 g/mol aufweist, gemessen wie in der Beschreibung angegeben.

7. Harz gemäß Anspruch 1, worin das Harz eine Glasübergangstemperatur (Tg) von 100°C bis 160°C aufweist, gemessen wie in der Beschreibung angegeben.

8. Harz gemäß Anspruch 1, worin der Brechungsindex des Harzes, der wie in der Beschreibung angegeben bei einer Wellenlänge von 589 nm gemessen wird, 1,64 bis 1,71 beträgt.

9. Verfahren zur Herstellung des Harzes gemäß irgendeinem der Ansprüche 1 bis 8, wobei das Verfahren umfasst:

Polymerisieren einer Zusammensetzung zur Herstellung eines Harzes, die eine durch die folgende chemische Formel 1a dargestellte Verbindung; und einen Polyestervorläufer oder einen Polycarbonatvorläufer umfasst:

[Chemische Formel 1a]

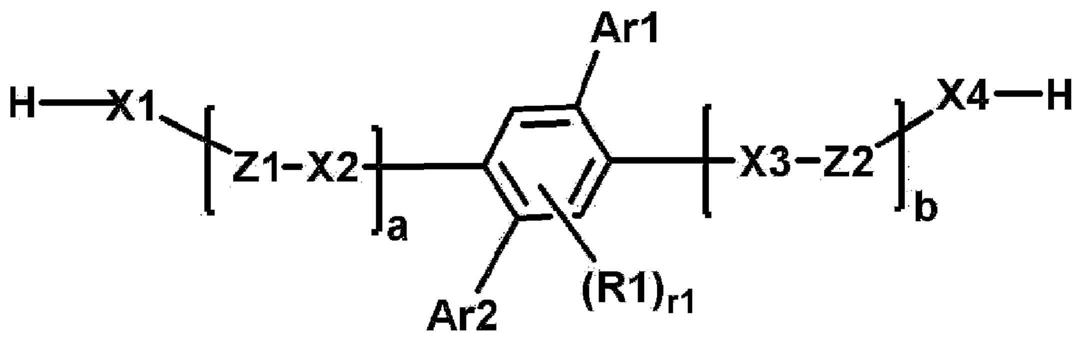

worin in der chemischen Formel 1a die Definitionen von Ar1, Ar2, R1, r1, X1 bis X4, Z1, Z2, a und b die gleichen sind wie die Definitionen in der chemischen Formel 1.

10. Verfahren gemäß Anspruch 9, worin der Polyestervorläufer durch die folgende chemische Formel A dargestellt ist und der Polycarbonatvorläufer durch die folgende chemische Formel B dargestellt ist:

[Chemische Formel A]

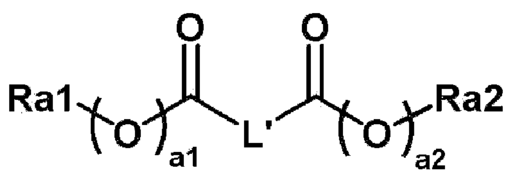

[Chemische Formel B]

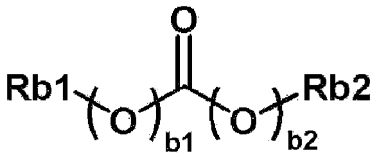

worin in den chemischen Formeln A und B

Ra1, Ra2, Rb1 und Rb2 gleich oder verschieden sind und jeweils unabhängig eine Halogengruppe; eine substituierte oder unsubstituierte Alkylgruppe; oder eine substituierte oder unsubstituierte Arylgruppe sind,
L' eine substituierte oder unsubstituierte Arylengruppe ist und
a1, a2, b1 und b1 jeweils 0 oder 1 sind.

**11.** Verbindung, dargestellt durch die folgende chemische Formel 1a:

[Chemische Formel 1a]

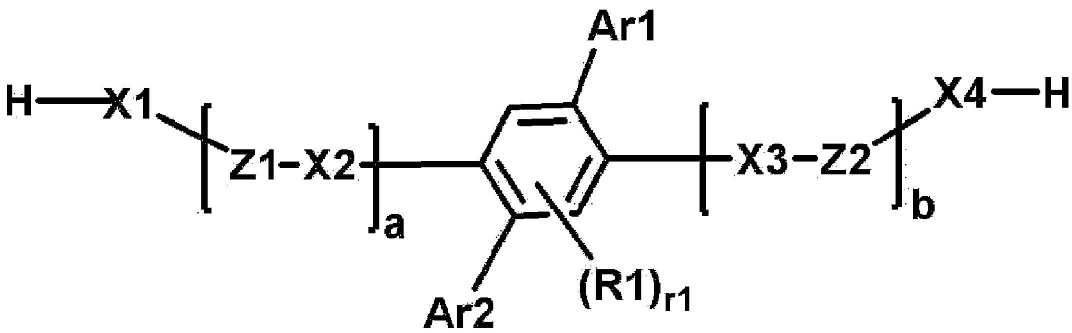

worin in der chemischen Formel 1a

Ar1 und Ar2 gleich oder verschieden sind und jeweils unabhängig eine Arylgruppe, die unsubstituiert ist oder substituiert ist mit einer substituierten oder unsubstituierten Alkylgruppe, einer substituierten oder unsubstituierten Arylgruppe, oder einer substituierten oder unsubstituierten Heteroarylgruppe; oder eine substituierte oder unsubstituierte polycyclische Heteroarylgruppe sind,
wenn Ar1 und Ar2 gleich sind und eine Phenylgruppe sind, die Phenylgruppe mit einer substituierten oder unsubstituierten Alkylgruppe, einer substituierten oder unsubstituierten Arylgruppe oder einer substituierten oder unsubstituierten Heteroarylgruppe substituiert ist,
R1 Wasserstoff; oder eine substituierte oder unsubstituierte Alkylgruppe ist,
r1 eine ganze Zahl von 0 bis 2 ist, und wenn r1 2 ist, zwei R1 gleich oder verschieden sind,
X1 bis X4 gleich oder verschieden sind und jeweils unabhängig O; oder S sind,
Z1 und Z2 gleich oder verschieden sind und jeweils unabhängig eine substituierte oder unsubstituierte Alkylengruppe; oder eine substituierte oder unsubstituierte Cycloalkylengruppe sind, und
a und b gleich oder verschieden sind und jeweils unabhängig eine ganze Zahl von 1 bis 10 sind, und wenn a und b jeweils 2 oder größer sind, die Strukturen in jeder Klammer gleich oder voneinander verschieden sind.

**12.** Harzzusammensetzung, umfassend das Harz gemäß irgendeinem der Ansprüche 1 bis 8.

**13.** Formartikel, umfassend die Harzzusammensetzung gemäß Anspruch 12.

**14.** Formartikel gemäß Anspruch 13, worin der Formartikel eine optische Linse ist.

## Revendications

1. Résine comprenant une unité représentée par la Formule chimique 1 suivante :

[Formule chimique 1]

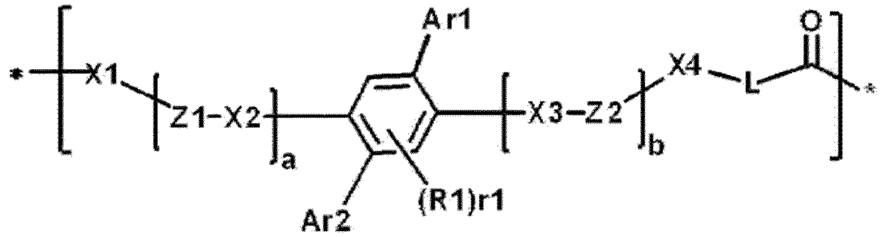

dans la Formule chimique 1,

Ar1 et Ar2 sont identiques l'un à l'autre ou différents l'un de l'autre, et sont chacun indépendamment un groupe aryle substitué ou non substitué ; ou un groupe hétéroaryle substitué ou non substitué,
R1 est un hydrogène ; ou un groupe alkyle substitué ou non substitué,
r1 est un nombre entier de 0 à 2, et lorsque r1 est égal à 2, deux R1 sont identiques l'un à l'autre ou différents l'un de l'autre,
L est une liaison directe ; ou -CO-L'-,
L' est un groupe arylène substitué ou non substitué,
X1 à X4 sont identiques les uns aux autres ou différents les uns des autres, et sont chacun indépendamment O ; ou S,
Z1 et Z2 sont identiques l'un à l'autre ou différents l'un de l'autre, et sont chacun indépendamment un groupe alkylène substitué ou non substitué ; ou un groupe cycloalkylène substitué ou non substitué, et
a et b sont identiques l'un à l'autre ou différents l'un de l'autre, et sont chacun indépendamment un nombre entier de 1 à 10, et lorsque a et b sont chacun supérieurs ou égaux à 2, les structures dans chaque parenthèse sont identiques l'une à l'autre ou différentes l'une de l'autre, et
* désigne un groupement lié à la chaîne principale de la résine.

2. Résine selon la revendication 1, dans laquelle R1 est un hydrogène.

3. Résine selon la revendication 1, dans laquelle Ar1 et Ar2 sont identiques l'un à l'autre ou différents l'un de l'autre, et sont chacun indépendamment un groupe aryle substitué ou non substitué présentant 6 à 12 atomes de carbone ; ou un groupe hétéroaryle substitué ou non substitué présentant 3 à 12 atomes de carbone.

4. Résine selon la revendication 1, dans laquelle Z1 et Z2 sont identiques l'un à l'autre ou différents l'un de l'autre, et sont chacun indépendamment un groupe alkylène substitué ou non substitué présentant 1 à 10 atomes de carbone.

5. Résine selon la revendication 1, dans laquelle X1 à X4 sont un O.

6. Résine selon la revendication 1, dans laquelle la résine présente un poids moléculaire moyen en poids de 10 000 g/mol à 200 000 g/mol, mesuré tel qu'indiqué dans la description.

7. Résine selon la revendication 1, dans laquelle la résine présente une température de transition vitreuse (Tg) de 100 °C à 160 °C, mesurée tel qu'indiqué dans la description.

8. Résine selon la revendication 1, un indice de réfraction de la résine, qui est mesuré à une longueur d'onde de 589 nm, est de 1,64 à 1,71 tel qu'indiqué dans la description.

9. Procédé de préparation de la résine selon l'une quelconque des revendications 1 à 8, le procédé comprenant :

une polymérisation d'une composition pour préparer une résine, qui comprend un composé représenté par la Formule chimique la suivante ; et un précurseur de polyester ou un précurseur de polycarbonate :

[Formule chimique 1a]

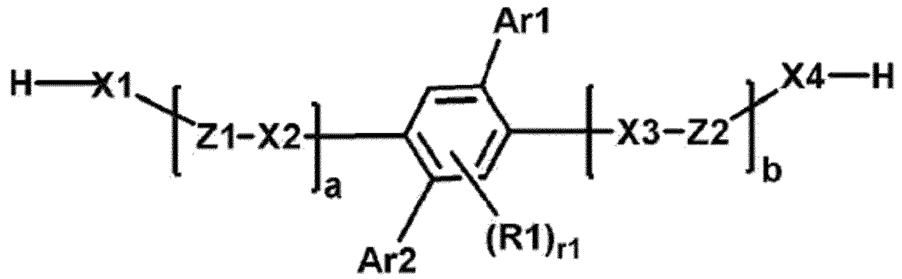

dans la Formule chimique 1a, les définitions de Ar1, Ar2, R1, r1, X1 à X4, Z1, Z2, a et b sont identiques aux définitions dans la Formule chimique 1.

**10.** Procédé selon la revendication 9, dans lequel le précurseur de polyester est représenté par la Formule chimique A suivante, et le précurseur de polycarbonate est représenté la Formule chimique B suivante :

[Formule chimique A]

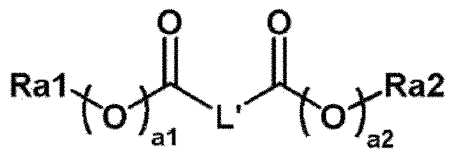

[Formule Chimique B]

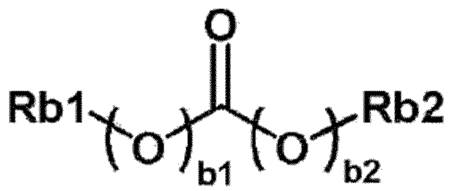

dans les Formules chimiques A et B,

Ra1, Ra2, Rb1 et Rb2 sont identiques les uns aux autres ou différents les uns des autres, et sont chacun indépendamment un groupe halogène ; un groupe alkyle substitué ou non substitué ; ou un groupe aryle substitué ou non substitué,
L' est un groupe arylène substitué ou non substitué, et
a1, a2, b1 et b2 sont chacun égaux à 0 ou 1.

**11.** Composé représenté par la Formule chimique 1a suivante :

[Formule chimique 1a]

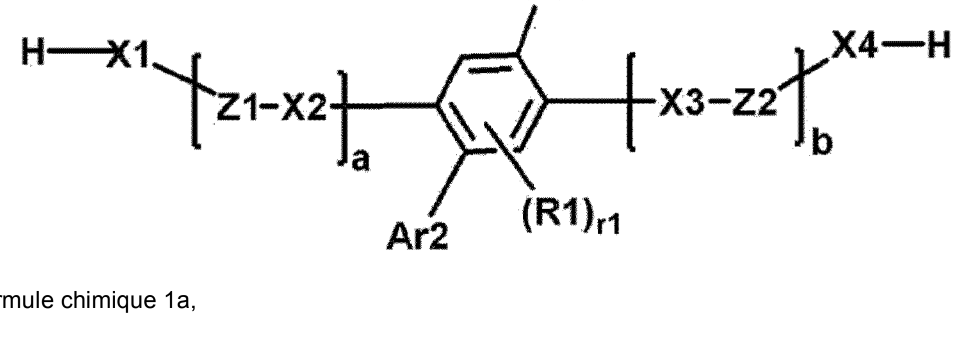

dans la Formule chimique 1a,

Ar1 et Ar2 sont identiques l'un à l'autre ou différents l'un de l'autre, et sont chacun indépendamment un groupe aryle qui est non substitué ou substitué par un groupe alkyle substitué ou non substitué, un groupe aryle substitué ou non substitué, ou un groupe hétéroaryle substitué ou non substitué ; ou un groupe hétéroaryle polycyclique substitué ou non substitué,
lorsque Ar1 et Ar2 sont identiques l'un à l'autre et sont un groupe phényle, le groupe phényle est substitué par un groupe alkyle substitué ou non substitué, un groupe aryle substitué ou non substitué, ou un groupe hétéroaryle substitué ou non substitué,
R1 est un hydrogène ; ou un groupe alkyle substitué ou non substitué,
r1 est un nombre entier de 0 à 2, et lorsque r1 est égal à 2, deux R1 sont identiques l'un à l'autre ou différents l'un de l'autre,
X1 à X4 sont identiques les uns aux autres ou différents les uns des autres, et sont chacun indépendamment O ; ou S,
Z1 et Z2 sont identiques l'un à l'autre ou différents l'un de l'autre, et sont chacun indépendamment un groupe alkylène substitué ou non substitué ; ou un groupe cycloalkylène substitué ou non substitué, et
a et b sont identiques l'un à l'autre ou différents l'un de l'autre, et sont chacun indépendamment un nombre entier de 1 à 10, et lorsque a et b sont chacun supérieurs ou égaux à 2, les structures dans chaque parenthèse sont identiques l'une à l'autre ou différentes l'une de l'autre.

**12.** Composition de résine comprenant la résine selon l'une quelconque des revendications 1 à 8.

**13.** Article moulé comprenant la composition de résine selon la revendication 12.

**14.** Article moulé selon la revendication 13, dans lequel l'article moulé est une lentille optique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020210063418
- WO 2004060962 A